# FASCICULE DE BREVET EUROPEEN

(11) **EP 4 103 244 B1**
(45) Date de publication et mention de la délivrance du brevet: **28.01.2026**
(21) Numéro de dépôt: 21704299.3
(22) Date de dépôt: 15.02.2021
(51) Int. Cl.: A61L 9/12, G06F 3/041

(54) **SYSTÈME DE SÉLECTION D'UNE FRAGRANCE**
SYSTEM ZUR AUSWAHL EINES DUFTSTOFFES
SYSTEM FOR SELECTING A FRAGRANCE

(30) Priorité: 14.02.2020 FR 2001460
(43) Date de publication de la demande: 21.12.2022
(73) Titulaire: Raimbault Semeraro, Audrey, Londres SW7 3JH (GB)
(72) Inventeur: Raimbault Semeraro, Audrey, Londres SW7 3JH (GB)
(74) Mandataire: Hautier IP
(86) Numéro de dépôt international: PCT/EP2021/053682
(87) Numéro de publication internationale: WO 2021/160892

(56) Documents cités:
- WO-A1-2018/163361

## Description

### DOMAINE TECHNIQUE

La présente invention concerne le domaine de la parfumerie, et plus spécifiquement. Elle trouve pour application particulièrement avantageuse le domaine de la sélection d'une fragrance.

### ETAT DE LA TECHNIQUE

Il existe un très grand nombre de parfums. De plus, ce nombre est en constante augmentation. Ainsi, il est de plus en plus difficile pour un utilisateur de sélectionner un parfum spécifique qui répond à ses attentes.

Un objet de la présente invention est donc de proposer un système et un procédé permettant d'aider un utilisateur à sélectionner le parfum qui répondra à ses attentes et, par exemple, en ayant un effet sur son comportement ou celui des personnes de son entourage.

Actuellement, ce sont principalement les vendeurs de parfums qui essayent d'aiguiller les utilisateurs dans le choix de la fragrance la plus adaptée, ce qui en fait un système peu fiable et basé exclusivement sur des aspects marketing des vendeurs, voire leurs objectifs commerciaux.

Ainsi, la présente invention a pour objectif d'offrir à l'utilisateur un système et un procédé améliorés d'aide à la sélection d'une fragrance par exemple en fonction de l'influence souhaitée et de son état émotionnel.

Le document WO2018/163361 divulgue un procédé de sélection d'un parfum en fonction d'une humeur à atteindre.

Le processus de ce document n'est pas satisfaisant en ce qu'il est limité tant dans l'éventail de solutions odorantes à fournir à l'utilisateur que dans les méthodes pour déterminer ladite solution odorante. Ainsi, il existe un besoin d'amélioration de la sélection de la fragrance divulguée dans le document WO2018/163361.

Les autres objets, caractéristiques et avantages de la présente invention apparaîtront à l'examen de la description suivante et des dessins d'accompagnement. Il est entendu que d'autres avantages peuvent être incorporés.

### RESUME

Pour atteindre cet objectif, selon un mode de réalisation on prévoit un système d'aide à la sélection par un utilisateur d'une fragrance dans une base de données comportant une pluralité de fragrances, dans lequel le système comprend :
- une interface utilisateur configurée pour recevoir et transmettre au système au moins une donnée relative à un objectif à atteindre et un profil utilisateur de l'utilisateur, la donnée relative à un objectif à atteindre et le profil utilisateur étant fonction de données personnelles fournies directement ou indirectement par l'utilisateur,
- un dispositif configuré pour générer au moins un stimulus destiné à un reçu par l'utilisateur,
- au moins un capteur physiologique configuré pour capter au moins une, de préférence deux, donnée physiologique de l'utilisateur en réponse audit au moins un stimulus le système étant configuré pour générer un profil physiologique à partir de l'au moins une donnée physiologique,
- le système étant configuré pour générer un profil physiologique à partir de l'au moins une donnée physiologique,
- et pour sélectionner au moins une fragrance utilisateur dans la base de données en fonction du profil physiologique, du profil utilisateur et de la donnée relative à un objectif à atteindre.

Comme indiqué précédemment, de nombreux parfums existent à ce jour. La détermination d'un parfum qui répond aux attentes d'un utilisateur n'est pas aisée et dépend de très nombreux paramètres. L'impact de la perception olfactive de l'utilisateur qui porte une fragrance sur son comportement ou celui de son entourage immédiat peut, par exemple, être l'amélioration d'une humeur, de la confiance en soi, d'une sérénité, d'une ouverture aux autres. Par ailleurs, les interactions entre les paramètres physiologiques de l'utilisateur, tel que par exemple non limitatif sa température, son odeur corporelle naturelle, sa sudation, etc. et les molécules de la fragrance peuvent modifier l'effet de la fragrance. Ces modifications d'effet peuvent être, par exemple, une modification de l'intensité, de la persistance, des allergies ou même de l'odeur de la fragrance. Ainsi, pour chaque utilisateur, en fonction de ces paramètres physiologiques et en fonction de la composition moléculaire de la fragrance, des effets chimiques différents peuvent apparaitre. L'invention permet ainsi de prendre en considération ces interactions chimiques afin de déterminer la fragrance ou un groupe de fragrance adéquat.

Un autre aspect de l'invention concerne un système d'aide à la sélection par un utilisateur d'une fragrance dans une base de données comportant une pluralité de fragrances, caractérisé en ce que le système comprend :
- une interface utilisateur configurée pour recevoir et transmettre au système au moins une donnée relative à un objectif à atteindre et un profil utilisateur de l'utilisateur, la donnée relative à un objectif à atteindre et le profil utilisateur étant fonction de données personnelles fournies directement ou indirectement par l'utilisateur,
- un dispositif configuré pour générer plusieurs stimuli destinés à un reçu par l'utilisateur, lesdits stimuli étant des stimuli olfactifs, visuels et/ou sonores,
- au moins deux capteurs physiologiques configurés pour capter au moins deux données physiologiques différentes de l'utilisateur en réponse auxdits stimuli,
- un dispositif configuré pour capter au moins une onde cérébrale générée par l'utilisateur en réponse auxdits plusieurs stimuli et pour générer au moins un signal cérébral en fonction de cette onde cérébrale, le signal cérébral étant différent des au moins deux données physiologiques, de préférence, les au moins deux données physiologiques captées par les capteurs physiologiques sont différentes de l'au moins une onde cérébrale;
- le système étant configuré pour générer un profil physiologique à partir des au moins deux données physiologiques et du au moins un signal cérébral,
et pour sélectionner au moins une fragrance utilisateur dans la base de données en fonction dudit profil physiologique généré, du profil utilisateur et de la donnée relative à un objectif à atteindre. De préférence mais non limitativement, le profil physiologique est généré à partir des au moins deux données physiologiques et du signal cérébral.

Un autre aspect concerne un procédé d'utilisation du système pour aider un utilisateur à la sélection d'une fragrance dans une base de données comportant une pluralité de fragrances comportant chacune un profil de fragrance, dans lequel le procédé d'aide à la sélection d'une fragrance comprend les étapes suivantes mises en œuvre par un système informatique à l'aide d'au moins un microprocesseur :
- Génération d'une donnée relative à un objectif à atteindre et d'un profil utilisateur de l'utilisateur, la donnée relative à un objectif à atteindre et le profil utilisateur étant fonction de données personnelles fournies directement ou indirectement par l'utilisateur;
- Génération d'une présélection de fragrances basée notamment sur la donnée relative à un objectif à atteindre, le profil utilisateur et sur des caractéristiques chimiques des fragrances stockées dans la base de données,
- Génération d'au moins un, de préférence au moins deux, stimulus destiné à un reçu par l'utilisateur,
- Génération d'un profil physiologique basé sur la génération et la captation d'au moins une, de préférence au moins deux, donnée physiologique de l'utilisateur en réponse audit au moins un stimulus;
- Détermination par un algorithme d'un profil de fragrance utilisateur en fonction du profil physiologique, du profil utilisateur et de la donnée relative à un objectif à atteindre de l'utilisateur;
- Comparaison du profil de fragrance utilisateur déterminé par l'algorithme avec les profils des fragrances de la base de données et sélection d'au moins une fragrance utilisateur;
- Affichage de l'au moins une fragrance utilisateur sélectionnée.

Optionnellement, le procédé d'utilisation comprend aussi une étape de génération d'un profil physiologique basé sur :
- au moins un signal cérébral, le signal cérébral étant basé sur au moins une onde cérébrale générée par l'utilisateur en réponse auxdits au moins deux stimuli;;
- la Génération et la captation d'au moins deux donnée physiologique de l'utilisateur différents de l'au moins une onde cérébrale, en réponse audit au moins deux stimulus;

Le procédé selon la présente invention permet la sélection de la fragrance selon les attentes de l'utilisateur. Cette sélection se fait notamment grâce à ses paramètres physiologiques et à son profil utilisateur. Cela permet de sélectionner une fragrance avec une bonne correspondance chimique avec l'utilisateur et qui répond à ses attentes. On entend ici par correspondance chimique la correspondance entre les différentes molécules de la fragrance avec les molécules du corps de l'utilisateur.

Enfin, l'invention concerne aussi une cabine comprenant un système comprenant, une interface utilisateur, un écran et au moins un capteur physiologique configuré pour capter une donnée physiologique.

L'invention concerne une cabine comprenant un système tel que décrit dans la présente demande et mettant en œuvre le procédé tel que décrit dans la présente demande, la cabine comprenant un casque neuronal configuré pour capter au moins une onde cérébrale, une interface utilisateur, un écran et au moins un capteur physiologique configuré pour capter les au moins deux données physiologiques différentes et différentes de l'onde cérébrale captée par le casque neuronal.

Un mode de réalisation de l'invention concerne un système d'aide à la sélection par un utilisateur d'une fragrance dans une base de données comportant une pluralité de fragrances, caractérisé en ce que le système comprend :
- une interface utilisateur configurée pour recevoir et transmettre au système au moins une donnée relative à un objectif à atteindre et un profil utilisateur de l'utilisateur, la donnée relative à un objectif à atteindre et le profil utilisateur étant fonction de données personnelles fournies directement ou indirectement par l'utilisateur,
- au moins un capteur physiologique configuré pour capter au moins une, de préférence deux, donnée physiologique de l'utilisateur,
- le système étant configuré pour générer un profil physiologique à partir de l'au moins une donnée physiologique,
- et pour sélectionner au moins une fragrance utilisateur dans la base de données en fonction du profil physiologique, du profil utilisateur et de la donnée relative à un objectif à atteindre.

### BREVE DESCRIPTION DES FIGURES

Les buts, les objets, ainsi que les caractéristiques et avantages de l'invention ressortiront mieux de la description détaillée d'un mode de réalisation de cette dernière qui est illustrée par les dessins d'accompagnement suivants dans lesquels :

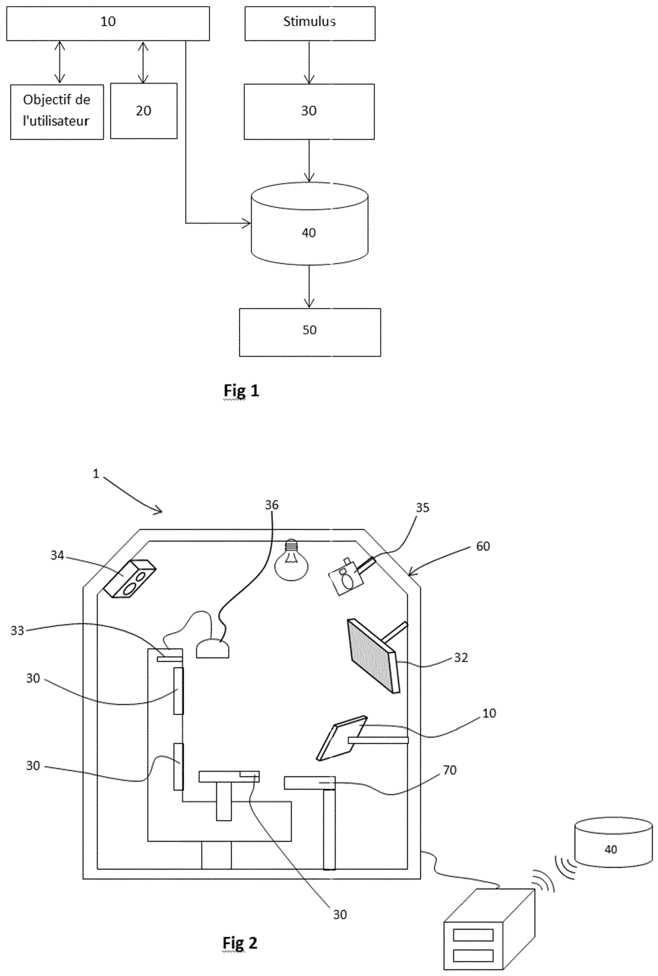

La figure 1 représente un schéma généraliste des principes de l'invention.
La figure 2 est un exemple de réalisation d'une cabine mettant en œuvre le système.
La figure 3 représente un schéma d'un exemple de réalisation plus précise du système selon l'invention.
La figure 4 représente une réalisation du processus d'aide à la sélection du parfum.
   Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques.

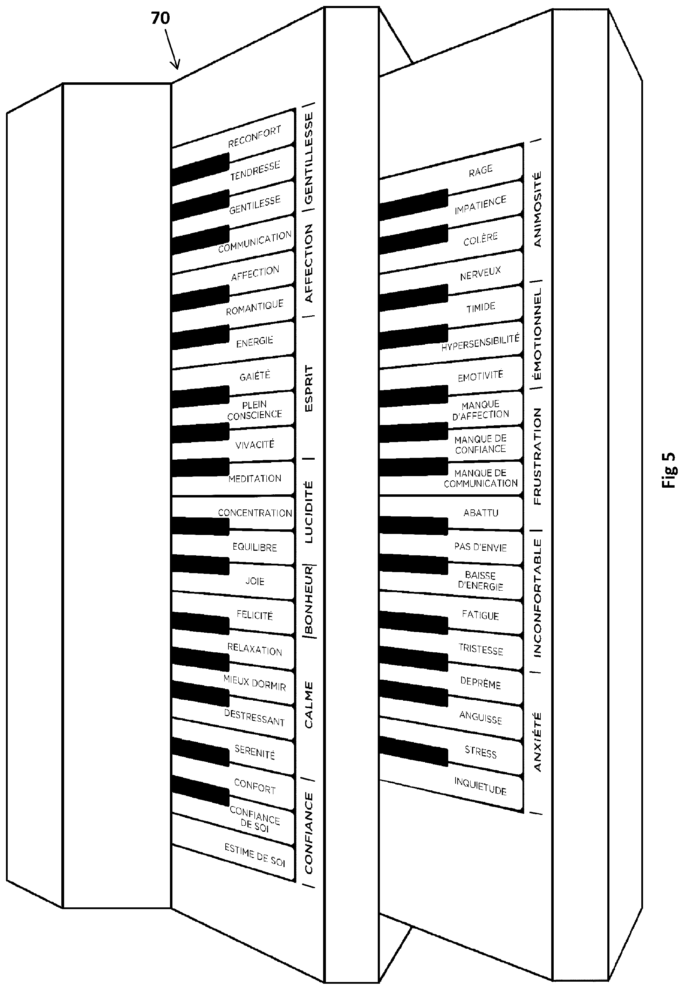

La figure 5 est une illustration d'une réalisation du dispositif d'interface émotionnelle

### DESCRIPTION DÉTAILLÉE

Avant d'entamer une revue détaillée des modes de réalisation de l'invention, sont énoncées ci-après des caractéristiques optionnelles qui peuvent éventuellement être utilisées en association ou alternativement :
- Le système comprend un dispositif configuré pour capter au moins une onde cérébrale de l'utilisateur et pour générer un signal cérébral de préférence en fonction d'au moins un stimulus de l'utilisateur, le signal cérébral étant différent de l'au moins une donnée physiologique, et dans lequel le profil physiologique est généré à partir au moins de l'au moins une donnée physiologique et à partir du signal cérébral.
- le profil physiologique est généré sans que l'utilisateur ne donne sciemment d'instructions en réponse auxdits au moins deux stimuli.

En particulier, l'utilisateur n'appuie sur aucun bouton ou n'émet aucun ordre ou réponse vocal en réponse auxdits au moins deux stimuli.

Ainsi le profil physiologique est généré sans que l'utilisateur n'intervienne ou ne confirme sciemment son ressenti.
- la fragrance utilisateur est sélectionnée sans que l'utilisateur ne donne sciemment d'instructions en réponse auxdits au moins deux stimuli.

En particulier, l'utilisateur n'appuie sur aucun bouton ou n'émet aucun ordre ou réponse vocal en réponse auxdits au moins deux stimuli. Ainsi la fragrance utilisateur est sélectionnée par le système sans aucun retour de la part de l'utilisateur.

Ainsi, l'invention permet que le processus de sélection de la fragrance ne soit pas altéré par l'état conscient de l'utilisateur. Le processus de sélection est lié à l'état inconscient et émotionnel de l'utilisateur.

Par conséquent, l'invention permet une sélection de fragrance qui soit plus efficace pour agir sur l'état émotionnel désiré.

Le profil utilisateur comprend au moins une donnée prise parmi l'âge, le sexe, le genre, le trait de caractère ou l'origine ethnique de l'utilisateur.
- Le profil utilisateur peut comprendre des données relatives à l'état émotionnel de l'utilisateur avant la réception d'au moins un stimulus par l'utilisateur.
- Le au moins un capteur physiologique est configuré pour capter à plusieurs et de préférence au moins deux, de préférence au moins trois, de préférence au moins quatre, de préférence au moins cinq, données physiologiques. Il est précisé que la précision du système peut être augmentée en prenant en considération un nombre plus important de données physiologiques.
- les au moins deux, données physiologiques différentes de l'onde cérébrale et capté par le capteur physiologique sont prise parmi la température, la tension artérielle, la sudation, l'émotion, le rythme respiratoire, la conductivité de la peau, la composition de la salive, le mouvement des yeux, la dilatation de la pupille ou le rythme cardiaque de l'utilisateur.

Le système comprend plusieurs capteurs configurés pour capter chacun une donnée physiologique différentes.
- Le au moins un capteur physiologique configuré pour capter l'au moins une, et de préférence plusieurs, donnée physiologique autre qu'une onde cérébrale est configuré pour capter au moins une donnée physiologique prise parmi la température, la tension artérielle, la sudation, l'émotion, le rythme respiratoire, la conductivité de la peau, la composition de la salive, le mouvement des yeux, la dilatation de la pupille ou le rythme cardiaque de l'utilisateur,
- Le au moins un capteur physiologique configuré pour capter l'au moins une, et de préférence plusieurs, donnée physiologique autre qu'une onde cérébrale est configuré pour capter toutes les données physiologiques suivantes : la température, la tension artérielle, la sudation, l'émotion, le rythme respiratoire, la conductivité de la peau, la composition de la salive, le mouvement des yeux, la dilatation de la pupille ou le rythme cardiaque de l'utilisateur.
- Chacune des fragrances de la base de données comprend un profil de fragrance distinct des autres fragrances, le système est, en outre, configuré pour déterminer un profil de fragrance utilisateur en fonction du profil physiologique, du profil utilisateur et de la donnée relative à un objectif à atteindre, et comprenant un algorithme configuré pour:
   - comparer le profil de fragrance utilisateur et le profil de chacune des fragrances de la base de données comportant une pluralité de fragrances et
   - sélectionner l'au moins une fragrance utilisateur en fonction de ladite comparaison.
- Le système comprend un dispositif d'interface émotionnelle configuré pour réaliser une interface entre l'utilisateur et le système et permettant à l'utilisateur d'indiquer des données relatives à son état émotionnel avant et/ou suite à l'émission des au moins deux stimuli. Le système comprend un microphone permettant d'une part de remplir les données du profil utilisateur grâce à la voix, et/ou d'autre part d'avoir la fonction du dispositif d'interface émotionnelle.
- le dispositif d'interface émotionnelle comprend une pluralité de touches, et dans lequel chaque touche est configurée pour indiquer un état émotionnel de l'utilisateur de préférence par la génération d'un stimulus tactile et/ou visuel différent pour chacune des touches.
- Le dispositif d'interface émotionnelle est configuré pour réaliser une interface entre l'utilisateur et le système et permettant à l'utilisateur d'indiquer son état émotionnel avant de recevoir un stimuli et son état émotionnel objectif.
- chacune des touches du dispositif d'interface émotionnelle comprend un revêtement tactile différent associé à un état émotionnel spécifique.
- des touches du dispositif d'interface émotionnelle comprennent une couleur différente associée un état émotionnel spécifique.
- chaque touche du dispositif d'interface émotionnelle comprend un dispositif permettant de générer au moins un stimulus additionnel auprès de l'utilisateur permettant audit utilisateur d'indiquer son état émotionnel.
- Le système compare l'au moins une donnée physiologique de l'utilisateur captée avant ledit au moins un stimulus et l'au moins une donnée physiologique de l'utilisateur captée après ledit au moins un stimulus afin de vérifier l'état émotionnel de l'utilisateur.
- Le système comprend un capteur optique configuré pour permettre une reconnaissance faciale de l'utilisateur et dans lequel le système est configuré pour déterminer, en fonction de la reconnaissance faciale, au moins l'une des données du profil utilisateur ou une donnée physiologique autre que l'onde cérébrale.
- le dispositif permettant de générer un stimulus destiné à être reçu par l'utilisateur est un revêtement unique de contact avec l'utilisateur, le revêtement étant configuré pour provoquer auprès de l'utilisateur un ressenti tactile unique.
- Le système est configuré pour, de préférence par l'intermédiaire d'un dispositif de reconnaissance faciale du système tel que par exemple une caméra ou un appareil photographique, déterminer en fonction de la reconnaissance faciale de l'utilisateur au moins l'un parmi l'âge de l'utilisateur, le sexe de l'utilisateur, le genre de l'utilisateur, l'émotion de l'utilisateur, l'origine ethnique, la température de l'utilisateur, la sudation de l'utilisateur.
- La génération par le système du profil physiologique à partir de l'au moins une donnée physiologique, et la sélection par ledit système d'au moins une fragrance utilisateur dans la base de données en fonction du profil physiologique, du profil utilisateur et de la donnée relative à un objectif à atteindre est réalisé par une série d'algorithmes formant une intelligence artificielle.
- L'interface utilisateur est configurée pour recevoir et transmettre au système au moins une donnée relative à un état émotionnel de l'utilisateur défini avant émission d'un stimulus et qualifié d'état émotionnel pré-stimulus.
- L'état émotionnel pré-stimulus peut par exemple être pris parmi l'un des états suivants : La colère, l'anxiété, la joie, la confiance, le stress, la fatigue etc.
- Le système comprend de préférence plusieurs et de préférence au moins trois capteurs physiologiques configurés pour capter au moins une et de préférence au moins trois, donnée physiologique de l'utilisateur en réponse audit au moins un stimulus.
- Lesdits au moins deux stimuli présentent plusieurs paramètres, de préférence pris parmi une nature de stimulus, une durée, une intensité, lesdits paramètres étant déterminés automatiquement en fonction au moins desdites données personnelles fournies directement ou indirectement par l'utilisateur et/ou en fonction des données physiologiques et du au moins un signal cérébral capté respectivement par les au moins deux capteurs physiologiques et le dispositif configuré pour capter l'au moins une onde cérébrale générée par l'utilisateur en réponse auxdits plusieurs stimuli;
- le profil physiologique est généré sans que l'utilisateur ne donne sciemment d'instructions en réponse auxdits au moins deux stimuli.
- la fragrance utilisateur est sélectionnée sans que l'utilisateur ne donne sciemment d'instructions en réponse auxdits au moins deux stimuli.
- les stimuli présentent plusieurs paramètres, de préférence pris parmi une nature de stimulus, une durée, une intensité, lesdits paramètres étant déterminés automatiquement en fonction au moins desdites données personnelles fournies directement ou indirectement par l'utilisateur et/ou par les données physiologiques et l'au moins un signal cérébral capté respectivement par les au moins deux capteurs physiologiques et le dispositif de capture de l'au moins une onde cérébrale.
- le profil physiologique comprend en outre un historique de réponses de l'utilisateur aux stimuli générés et destinés à être reçus par l'utilisateur.
- ledit historique est généré en effectuant les étapes suivantes :
   - Identification, en fonction des objectifs à atteindre et de données personnelles, d'un profil cible préenregistré dans la base de données,
   - Au moins un cycle, et de préférence au moins deux cycles, chaque cycle comprenant les étapes successives suivantes, certains au moins de ces étapes étant effectuées par un système informatique de traitement de données:
      a. Génération d'au moins un stimulus additionnel,
      b. Captation d'au moins une donnée physiologique et d'un signal cérébral issus d'une onde cérébrale de l'utilisateur, la donnée physiologique et l'onde cérébrale étant générées par l'utilisateur en réponse audit au moins un stimulus additionnel,
      c. Actualisation du profil physiologique en fonction des données physiologiques et de l'onde cérébrale générée par l'utilisateur en réponse audit au moins un stimulus additionnel,
      d. Comparaison du profil physiologique actualisé avec des profils préenregistrés dans la base de données,
   Si le profil physiologique actualisé:
   - correspond à un profil préenregistré dans la base de données, alors génération de l'historique sur la base des stimuli additionnels de tous les cycles,
   - ne correspond à aucun profil préenregistré dans la base de données, alors répétition des étapes a à d avec des stimuli présentant des paramètres différents d'un cycle à l'autre.
- La génération de l'historique comprend au moins deux et de préférence au moins cinq cycles.

La cabine comprend un casque neuronal configuré pour capter une donnée physiologique différente de la donnée physiologique.
- La cabine comprend un système de diffusion d'un parfum par micro-nébulisation et/ou par diffusion sèche.

Il est précisé, dans le cadre de la présente invention, que les termes fragrance et parfum sont des synonymes et désignent tous deux une odeur.

Le système 1 selon l'invention permet d'aider un utilisateur à sélectionner un parfum en fonction de son objectif, de son profil utilisateur 20 et d'au moins une donnée physiologique 31 de préférence en réponse à au moins un stimulus.

Le système 1 selon l'invention permet d'aider un utilisateur à sélectionner une fragrance notamment en fonction des objectifs de l'utilisateur.

En effet, pour un même utilisateur, des parfums différents peuvent être portés selon les différents objectifs à atteindre. On entend ainsi par objectif ou attente à atteindre un effet psycho-physiologique ou olfactif sur le comportement de l'utilisateur portant la fragrance ou sur son entourage immédiat. Par exemples non limitatifs, cela peut être un trait de caractère à accentuer, une humeur à communiquer, une séduction dans un cadre romantique, un ressenti d'expérience dans le cadre d'un rendez-vous professionnel, ou encore la modification de la perception de son âge, etc. L'effet de la fragrance est ponctuel et à destination de personnes saines. Ainsi, ni le système 1 ni le procédé ne concernent la réalisation d'un traitement à but thérapeutique ou même d'une prévention thérapeutique.

L'objectif à atteindre peut être représenté par une donnée, qualifiée de donnée relative à un objectif à atteindre.

La prise en compte de l'objectif de l'utilisateur dans la détermination de la fragrance adéquate est primordiale pour une satisfaction optimale de l'utilisateur.

Ainsi, en fonction des objectifs à atteindre, différentes fragrances peuvent être utilisées. Le système 1 est configuré pour prendre en considération la donnée relative à un objectif à atteindre et filtrer certaines gammes de parfum dans la base de données 40 en fonction dudit objectif.

Pour ce faire, le système 1 comprend une interface utilisateur 10 permettant à l'utilisateur de renseigner son objectif. L'interface utilisateur 10 peut, par exemple, être un écran tactile, ou encore un clavier permettant de saisir des informations.

L'interface utilisateur 10 peut, par exemple, comprendre une liste de différents objectifs dans laquelle l'utilisateur doit sélectionner l'objectif qui lui sied le plus. L'avantage de cette réalisation est qu'il est plus aisé de lier certains profils de fragrances aux différents objectifs. En effet, en ayant des objectifs prédéterminés, il est possible d'associer à chacun des objectifs certains profils de fragrance.

Un profil de fragrance est associé à chaque fragrance stockée dans la base de données 40 comprenant plusieurs fragrances. Le profil de fragrance comprend notamment son odeur et les molécules la composant.

Par exemple, un certain type de molécules odorantes peuvent être ainsi associées à certains traits du profil utilisateur 20 de l'utilisateur. L'association de paramètres physiques de la fragrance, comme son type de molécules odorantes à un objectif déterminé est basé soit sur un système d'apprentissage, soit sur des bases de données existantes d'études scientifiques et/ou marketing, soit sur une combinaison des deux. La base de données peut être une base de données aromachologiques.

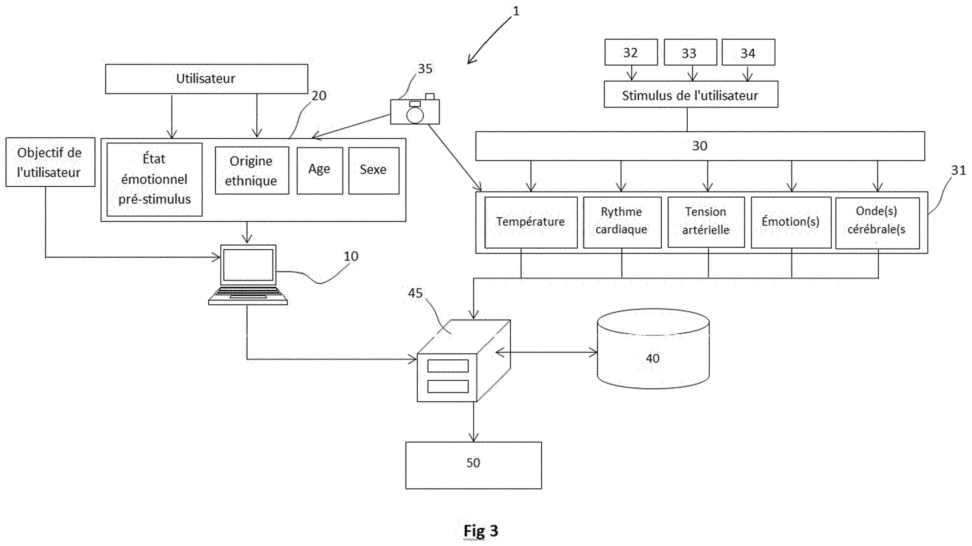

De préférence, le système 1 a accès à une base de données 40 comprenant pour chaque fragrance des données d'études scientifiques et commerciales. Avantageusement, la connexion avec la base de données 40 peut se faire par des moyens de communication sans fil tels qu'illustrés dans la figure 3, mais peut aussi être réalisée par des moyens de communication filaire (non visibles sur les figures). Ces études permettent d'avoir un retour sur les différents profils de fragrances en fonction des objectifs recherchés. De plus, le système 1 est configuré pour pouvoir enregistrer chaque sélection et apprendre, en fonction des retours utilisateurs, de la pertinence de l'association d'un profil de fragrance avec un objectif prédéterminé.

Dans d'autres réalisations de l'invention, l'utilisateur renseigne librement l'objectif qu'il souhaite atteindre. Dans ce cas, le système 1 comprend un sous-système permettant une correspondance entre l'objectif librement renseigné par l'utilisateur et les profils de fragrance disponibles dans la base de données comprenant plusieurs fragrances. Avantageusement, la correspondance se fait par le biais d'un algorithme d'apprentissage d'intelligence artificielle 45.

L'intelligence artificielle 45 est notamment mis en œuvre par au moins un microprocesseur L'algorithme est ainsi configuré pour apprendre en fonction des différents utilisateurs, de leurs différentes réponses physiologiques à différents stimulus quels sont les éléments permettant de réaliser les objectifs de l'utilisateur. Un apprentissage par le système est préférable, voire nécessaire afin de prendre en considération ses spécificités. Il est aussi possible de permettre à l'intelligence artificielle 45 d'apprendre les comportements génériques. Un tel apprentissage permet de faciliter la détermination et l'adaptation des spécificités de l'utilisateur. Cette réalisation, et l'apprentissage par l'algorithme permettent une plus grande finesse dans la formulation de l'objectif et la détermination de la fragrance pour l'utilisateur et de préférence une fragrance optimale.

L'apprentissage est notamment réalisé en partant des données physiologiques de l'utilisateur avant l'émission de tout stimulus et en le comparant avec un profil physiologique cible. Le profil physiologique cible étant fonction d'un objectif à atteindre et associé à l'état physiologique idéal et théorique que l'utilisateur doit atteindre pour remplir ledit objectif. Par exemple, l'état physiologique cible comprend un niveau de rythme cardiaque spécifique, une sudation spécifique, etc. en fonction de l'objectif à atteindre comme par exemple, un état reposé ou dynamisé. La comparaison entre l'état physiologique initial et l'état physiologique cible permet au système de définir quels sont les stimuli à générer. À chaque génération de stimuli, le système va capter les données physiologiques de l'utilisateur ainsi que ses ondes cérébrales. Cette captation va permettre au système d'apprendre la réaction de l'utilisateur, par le biais de ses données physiologiques et d'ondes cérébrales à chaque stimulus émis. L'apprentissage de ces réactions va permettre ensuite de réaliser un historique permettant d'améliorer le profil physiologique 31 de l'utilisateur. Il est à noter que ce profil évolue rapidement en fonction de nombreux paramètre. Ainsi un profil peut être pertinent à un instant T mais non pertinent à un instant T+1, notamment en fonction de l'état physiologique et émotionnel initial de l'utilisateur.

L'apprentissage est important, car en se basant uniquement sur un profil physiologique cible préenregistré, c'est-à-dire purement théorique, on ne peut pas sélectionner avec précision la fragrance pertinente pour réaliser un objectif. Il est donc important que le système possède la capacité d'apprendre les réactions de l'utilisateur à chaque stimulus qu'il émet à sa destination. Les étapes de génération de cet historique sont détaillées plus en amont de la description.

Pour rappel, un parfum ou une fragrance est la combinaison d'une ou plusieurs molécules odorantes, éventuellement dans un solvant. Les molécules odorantes vont ensuite entrer en contact et réagir avec l'utilisateur. Une interaction chimique entre la ou les molécules composant le parfum et les molécules de l'utilisateur est réalisée. Cette réaction chimique pouvant de plus avoir un effet comportemental différent chez les utilisateurs, par exemple avec une sensation de gêne, ou de bien-être, etc. De ce fait, en fonction des utilisateurs, une réaction différente du parfum peut intervenir.

Pour prendre en considération ces variations, et pour mieux pouvoir s'adapter à l'utilisateur, le système 1 génère et prend en considération le profil utilisateur 20 de l'utilisateur.

Le profil utilisateur 20 est ainsi généré en prenant en compte plusieurs paramètres par exemple, l'âge, le sexe, le genre, la présence ou non d'une peau atopique, ou encore l'origine ethnique ou les traits de caractère de l'utilisateur. La variation de sélection de la fragrance en fonction de ces paramètres est préférable afin d'avoir une fragrance en adéquation avec le profil utilisateur 20 de l'utilisateur. En effet, les paramètres par exemple, le sexe, l'âge ou l'origine ethnique de l'utilisateur impliquent la génération par le corps de molécules spécifiques en fonction des utilisateurs. Ces molécules interagissent chimiquement avec les molécules composantes de la fragrance. Il est ainsi possible de déterminer à l'avance, en fonction de ces paramètres, les interactions qui se réaliseront. La détermination de ces interactions permet alors de déterminer une ou plusieurs fragrances utilisateurs préférentielles.

On notera, par exemple, que certaines fragrances sont développées pour être adaptées à des personnes plus jeunes ou aux différents sexes, aux différentes origines ethniques ou encore en fonction du lieu d'utilisation du parfum. En effet, en fonction du lieu d'utilisation, l'environnement va venir altérer la composition chimique du parfum et/ou la transmission des molécules odorantes. Ces différences de profil utilisateur 20 de fragrance prennent en compte notamment les spécificités physiologiques de ces critères. De même, en fonction des traits de caractère tels que, par exemple, la timidité ou l'excentricité, certains types de parfum plairont ou non à l'utilisateur. Il est important que la fragrance plaise à l'utilisateur afin de pouvoir permettre à ce dernier d'atteindre son objectif. En effet, un parfum ne plaisant pas à son utilisateur lui causera un stress supplémentaire qui, bien souvent, sera un frein à l'atteinte de l'objectif fixé.

La prise en compte de ce profil utilisateur 20 permet d'améliorer la sélection du parfum en attente avec les spécificités de l'utilisateur.

Ainsi, comme indiqué ci-dessus, le système 1 prend en compte ces spécificités pour permettre de répondre à l'objectif fixé par l'utilisateur.

Par exemple, dans le cadre de la sélection d'un parfum pour une personne qui débute dans la vie active, la détermination de son âge peut permettre de sélectionner une fragrance qui renvoie un ressenti de maturité afin de compenser une apparence de manque d'expérience. La prise en compte de ces critères permet ainsi d'affiner grandement la sélection du parfum.

La prise en compte du profil utilisateur 20 peut être réalisée au travers de l'interface utilisateur 10. Dans cette réalisation, l'utilisateur répond, par exemple, à un questionnaire au travers d'un écran tactile par le biais d'un clavier. Dans une autre réalisation de l'invention, le système 1 comprend, en outre, un capteur optique 35 qui permet, au moyen d'un sous-système, une reconnaissance faciale. Le capteur optique est, de préférence, une caméra ou un appareil photographique. Le capteur peut capter de la lumière dans différentes gammes d'ondes lumineuses, y compris dans des gammes de lumière non visibles. Ainsi, cette reconnaissance faciale permet de déterminer automatiquement au moins l'un des paramètres et de préférence tous les paramètres suivants : le sexe, le genre, l'âge, les émotions, les mouvements oculaires, la dilatation de la pupille de l'œil, la température, la sudation, le rythme cardiaque ou encore l'origine ethnique de l'utilisateur.

Avantageusement, ce système permet une saisie automatique d'un nombre de paramètres importants. Dans ce mode de réalisation, l'utilisateur peut, si nécessaire, valider et/ou corriger les informations pré-remplies par le système 1 au travers de l'interface utilisateur 10.

Le système 1 peut aussi comprendre un module de connexion à un ou plusieurs serveurs externes sur lequel des données du profil utilisateur 10 sont déjà renseignées, tel que, par exemple, un réseau social de l'utilisateur. Ainsi, lors de l'utilisation, l'utilisateur peut se connecter à l'un de ses réseaux sociaux ce qui permet un pré-remplissage automatique du profil utilisateur 20. Le système 1 peut aussi prendre en compte diverses informations du réseau social afin de déterminer le profil général de l'utilisateur, notamment ses réactions en fonction des situations. Ce traitement de données permet, par exemple, de faciliter la reconnaissance faciale en déterminant, sur la base de diverses photographies publiées sur le réseau social, les caractéristiques de certaines émotions (traits du visage tels que la forme des lèvres, des yeux ou encore des pommettes lors d'un sourire, d'une grimace etc.). Cela peut aussi permettre de déterminer les lieux principaux de l'utilisateur grâce aux données de géolocalisation collectées par le réseau social. Ces données de géolocalisation permettent ainsi d'affiner la sélection du parfum en fonction des lieux géographiques d'utilisation principale du parfum.

Avantageusement, la reconnaissance faciale est configurée pour détecter au moins deux émotions différentes de l'utilisateur lorsqu'il est soumis à au moins un stimulus. De préférence, la reconnaissance faciale est configurée pour reconnaitre au moins quatre émotions différentes. De préférence, la reconnaissance faciale est configurée pour reconnaitre au moins six émotions différentes.

Comme indiqué précédemment, le système 1 d'aide à la sélection d'une fragrance se fait aussi en fonction d'au moins une donnée physiologique 31 en réponse à au moins un stimulus. Avantageusement, la détermination de la fragrance utilisateur 50 est réalisée en fonction d'au moins deux données physiologiques 31 en réponse à au moins un stimulus. Ces données physiologiques 31 permettent de déterminer un profil physiologique de l'utilisateur. De préférence, la détermination de la fragrance utilisateur 50 se fait sur une pluralité de données physiologiques afin d'avoir une finesse de détection des émotions de l'utilisateur améliorée, voire optimale.

Le système peut comprendre un microphone. La présence d'un microphone peut permettre d'une part, de remplir les informations du profil utilisateur 20 grâce à la voix et d'autre part cela permet aussi à l'utilisateur de renseigner des données relatives à son état émotionnel ou autrement dit son état émotionnel. En fonction de la variation de la voix (tremblement, assurance, etc.) en fonction des différents stimuli le système 1 est configuré pour déterminer l'état émotionnel de l'utilisateur.

Avantageusement, le profil utilisateur 20 comprend aussi des données relatives à l'état émotionnel de l'utilisateur avant toute émission de stimulus. Le renseignement de cet état émotionnel peut être fait soit par l'utilisateur directement au travers de l'interface utilisateur 10, soit en captant une ou plusieurs, c'est à dire au moins deux, de ces données physiologiques 30, soit en combinant ces deux modes de collectes de données. Le renseignement des données relatives à l'état émotionnel de l'utilisateur avant l'émission de stimulus permet de confirmer la variation de ces données physiologiques 31 et d'établir ainsi plus facilement le profil physiologique 31.

Dans une réalisation de l'invention, au moins l'une des données physiologiques 31 est une onde cérébrale captée par un capteur physiologique 30, de préférence un capteur cérébral comportant, de préférence, au moins deux électrodes telles que, par exemple, un casque neuronal 36. Dans une autre réalisation, le capteur cérébral est un dispositif auriculaire configuré pour capter des ondes cérébrales. Avantageusement, le système 1 capte plusieurs ondes cérébrales afin de générer un ou plusieurs signal cérébral tel que, par exemple, un électroencéphalogramme, en réponse à au moins un stimulus. Le capteur cérébral est configuré pour capter au moins l'une des données et de préférences plusieurs données parmi les ondes cérébrales thêta, alpha, bêta, gamma. Avantageusement, le capteur neuronal permet la création d'une interface cerveau/machine afin de surveiller l'évolution de l'activité cérébrale de l'utilisateur pendant le ou les stimuli.

Avantageusement, le système 1 est configuré pour interpréter le signal cérébral reçu en réponse à au moins un stimulus. Cette interprétation permet au système 1 de déterminer le ressenti de l'utilisateur à l'au moins un stimulus. Ainsi, le système 1 est capable de déterminer la réaction ou l'émotion de l'utilisateur suscitée par le stimulus. Autrement dit, cela permet au système 1 de déterminer si le stimulus est pour l'utilisateur plaisant, désagréable, euphorisant, etc.

Avantageusement, le casque neuronal 36 comprend entre deux et quinze électrodes.

L'état émotionnel de l'utilisateur en réponse au stimulus n'est pas uniquement déterminé par son signal cérébral tel que, par exemple, son électroencéphalogramme, mais aussi par les autres données physiologiques captées par le système 1 et/ou par les données issues de la reconnaissance faciale si elle est disponible. Avantageusement, le signal cérébral n'est pas obligatoire et la détection des émotions de l'utilisateur peut se faire par l'assimilation d'autres données physiologiques.

Par exemple, le système 1 peut comprendre au moins un capteur physiologique, et de préférence plusieurs capteurs physiologiques et de préférence au moins trois capteurs physiologiques. Par souci de concision, les capteurs physiologiques sont également désignés capteurs. Les capteurs sont par exemple pris parmi un capteur de température, un capteur de tension artérielle, un capteur de sudation, un capteur d'émotions, un capteur de rythme respiratoire, un capteur de conductivité électrique de la peau ou du rythme cardiaque de l'utilisateur. Le capteur peut aussi par exemple capter le mouvement des yeux ou la dilatation de la pupille. Dans une autre réalisation compatible avec les précédentes, le capteur peut permettre de récolter et d'analyser la salive de l'utilisateur. La captation de la salive permet notamment une analyse du niveau de stress de l'utilisateur. L'analyse du niveau de stress de l'utilisateur est notamment réalisée par la prise en compte des niveaux de cortisol contenu dans la salive. De plus, la collecte de la salive peut optionnellement permettre la réalisation d'une analyse ADN de l'utilisateur. Plus particulièrement, une analyse de certaines portions du génome en relation avec les données biologiques de l'utilisateur et/ou ses capacités olfactives. Cette analyse génétique peut ainsi permettre une meilleure sélection de la fragrance.

En fonction de l'ensemble des données récoltées, le système 1 est capable d'éditer et d'affiner le profil physiologique de l'utilisateur. Plus le nombre et le type de données sont importants, plus le profil physiologique 31 de l'utilisateur en fonction du stimulus sera précis. Cette précision accrue permet notamment, en combinaison avec le profil utilisateur 20, de déterminer précisément le profil de fragrance utilisateur 50 pour l'utilisateur.

Dans une réalisation de l'invention, les capteurs physiologiques peuvent être portés par l'utilisateur en dehors du système 1. C'est, par exemple, le cas du capteur de rythme cardiaque, par exemple, qui peut être une montre connectée. Plus généralement, plusieurs données physiologiques peuvent être captées par des objets connectés portés par l'utilisateur, tels que, par exemple, un téléphone intelligent, un bracelet connecté, un podomètre, un stimulateur cardiaque, etc. Dans cette réalisation, le système 1 comprend un dispositif de communication, de préférence sans fil tel que, par exemple, du Wifi^{®}, du Bluetooth^{®}, un réseau téléphonique, etc., avec l'appareil connecté porté par l'utilisateur. Cette connexion entre le système 1 et la montre connectée permet au système 1 de récupérer au moins une et de préférence toutes les données physiologiques enregistrées par la montre connectée. Avantageusement, la reprise de l'historique de certaines données physiologiques de l'utilisateur par le système 1 lui permet de mieux appréhender l'état émotionnel quotidien de l'utilisateur. Cette prise en compte peut permettre de préciser les objectifs de l'utilisateur et les moyens de les atteindre.

Le système 1 est configuré pour intégrer les données physiologiques, de préférence, après une stimulation de l'utilisateur.

Pour ce faire, le système 1 comprend au moins un dispositif configuré pour générer au moins un stimulus et de préférence plusieurs (c'est-à-dire au moins deux) stimuli. Ce stimulus est destiné à être reçu par l'utilisateur. De préférence, l'au moins un dispositif est configuré pour émettre plusieurs, c'est à dire au moins deux, stimuli à destination de l'utilisateur. Les types de stimuli peuvent être des odeurs, des images, des sons ou encore des variations de température. Les types de stimuli peuvent être tous différents ou tous les mêmes ou encore une combinaison de type de stimuli identique et différents. De préférence, les stimuli sont des signaux olfactifs tels que, par exemple, des odeurs et/ou des signaux visuels tels que, par exemple, des images. Les stimuli peuvent être émis respectivement successivement ou concomitamment par un ou plusieurs écrans 32, un ou plusieurs diffuseurs d'odeur 33 et/ou une ou plusieurs enceintes 34 ou encore grâce à un casque de réalité virtuelle et des écouteurs (non représentés sur les figures). Cette dernière réalisation avec un casque permet une immersion complète de l'utilisateur et ainsi une meilleure réponse aux stimuli. Ainsi, une fois que le système 1 a déterminé le profil utilisateur 20, il peut réaliser une présélection d'une pluralité de fragrances de la base de données comportant plusieurs fragrances. Par cette présélection, le système 1 peut stimuler l'utilisateur afin de déterminer sa réaction à ces différentes fragrances présélectionnées. En fonction des réactions de l'utilisateur, le système 1 est ensuite capable de déterminer le profil physiologique 31 de l'utilisateur et d'affiner la sélection de fragrance.

Dans une réalisation de l'invention, le système 1 comprend un dispositif d'interface émotionnelle 70 permettant à l'utilisateur de renseigner son état émotionnel. Par exemple, ce dispositif d'interface émotionnel peut être un microphone, non représenté sur les figures. Ainsi, l'utilisateur peut renseigner son état émotionnel grâce à sa voix. Le dispositif d'interface émotionnelle 70 peut être physique ou virtuel. Dans une réalisation de l'invention, le dispositif prend la forme d'un questionnaire physique ou virtuel. À chaque stimulus, l'utilisateur peut renseigner son ressenti émotionnel par le biais, par exemple, de boutons physiques, tactiles ou virtuels et/ou de cases à cocher ou d'un curseur à déplacer. Cette dernière réalisation permet une plus grande finesse dans le choix du ressenti émotionnel.

Avantageusement, le dispositif d'interface émotionnelle 70 prend la forme d'un piano.

Le dispositif d'interface émotionnelle 70 comprend une pluralité de touches. Chacune des touches est associée à une émotion. Les touches sont, de préférence, regroupées entre elles par catégorie d'émotions, par exemple l'anxiété, le bien-être, l'animosité, l'inconfort, le confort, l'affection et la douceur. Le regroupement des touches en fonction de grandes catégories d'émotions facilite la perception par l'utilisateur et donc sa faculté d'exprimer ses émotions.

Dans une réalisation de l'invention, chaque touche est configurée pour susciter une émotion spécifique de la part de l'utilisateur lorsque ce dernier la touche.

Dans la réalisation dans laquelle le dispositif d'interface émotionnelle 70 est un dispositif d'interface émotionnelle 70 physique, chaque touche comprend un revêtement différent. Chaque revêtement comprend un ressenti tactile différent par le biais, par exemple, d'une rugosité, d'une douceur ou d'aspérités différentes. Un faible courant électrique peut aussi parcourir les touches afin d'amplifier le ressenti tactile de l'utilisateur. Dans le cas dans lequel le dispositif d'interface émotionnelle 70 est un dispositif d'interface émotionnelle 70 virtuel, un retour haptique spécifique qui permet d'avoir un ressenti tactile au travers d'un écran par exemple. Le retour haptique peut être réalisé, par exemple, par une vibration localisée, un courant électrique ou toute autre méthode permettant un retour tactile à l'utilisateur.

Chacune des touches est associée à une émotion. Ainsi, lorsque l'utilisateur ressent un stimulus, il va pouvoir parcourir les touches et déterminer ensuite, grâce au ressenti tactile des touches, laquelle des touches correspond le mieux à son ressenti. Le dispositif d'interface émotionnelle 70 peut donc permettre d'identifier rapidement l'émotion ressentie par l'utilisateur. Dans la réalisation préférée de l'invention, le dispositif d'interface émotionnelle 70 peut permettre à l'utilisateur d'indiquer son état émotionnel pré-stimuli, c'est-à-dire avant qu'un stimulus soit généré à l'attention de l'utilisateur. Le dispositif d'interface émotionnelle 70 peut aussi permettre à l'utilisateur d'indiquer l'objectif d'état émotionnel à atteindre.

Dans une réalisation spécifique, le dispositif d'interface émotionnelle 70 peut être utilisé par l'utilisateur en réponse aux stimuli. Cependant, cette réalisation n'est pas obligatoire, notamment du fait qu'elle oblige l'utilisateur à intervenir lors du processus de détermination du profil physiologique 31. En effet, il est préférable que l'utilisateur soit parfaitement relaxé pendant la détermination du profil physiologique 31. L'usage de l'interface émotionnelle 70 lors de cette détermination du profil physiologique 31 nécessite une intervention de l'utilisateur, et donc un arrêt dans son processus de relaxation. De fait, le dispositif d'interface émotionnelle 70 peut ne pas être actif lors de la génération du profil physiologique.

Les touches du dispositif d'interface émotionnelle 70 peuvent être unicolores ou avoir une couleur différente les unes des autres. Avantageusement, le fait d'avoir une couleur spécifique par touche permet d'accentuer l'émotion associée à la touche.

Dans une autre réalisation de l'invention, les touches du dispositif d'interface émotionnelle 70 ont toutes un ressenti tactile identique. Dans une autre réalisation, compatible avec les précédentes, chaque touche émet un stimulus olfactif et/ou visuel distinct les uns des autres. Dans cette réalisation spécifique, l'émission de stimulus par l'interface émotionnelle 70 est de préférence réalisée en fin de processus de sélection et après la détermination du profil physiologique. Ces stimuli ont notamment pour objectif de vérifier la réaction psycho-physiologique de l'utilisateur aux fragrances sélectionnées après l'apprentissage du comportement de l'utilisateur par l'algorithme d'intelligence artificielle 45. Cette réalisation permet ainsi de valider la bonne connaissance de l'utilisateur par le système 1 et ainsi la sélection de fragrances faite par le système 1.

L'invention concerne aussi l'apprentissage par le système des réactions de l'utilisateur aux stimuli. Pour ce faire, la génération du profil physiologique 31 va s'actualiser après chaque stimulus émis par le système 1. En effet, un premier profil physiologique 31 est généré dès réception des données physiologiques captées par les capteurs physiologiques (30) et du signal cérébral capté par le dispositif configuré pour capter l'au moins une onde cérébrale, c'est à dire de préférence un casque neuronal. Le ou les autres stimuli ensuite émis par le système à l'attention de l'utilisateur permettent de capter d'autres données physiologiques et ondes cérébrales. Ces autres données sont ainsi implémentées dans le premier profil physiologique 31 afin de l'actualiser. Plusieurs actualisations du profil physiologique 31 sont réalisées avant la détermination de la fragrance utilisateur. Il est préférable, voire nécessaire, que cette apprentissage soit réalisé sans que l'utilisateur ne donne sciemment un retour au système afin de pouvoir sélectionner une fragrance utilisateur en parfaite adéquation avec la réponse du corps de l'utilisateur sans prise en compte de ses préjugés et préférences psychologiques.

Bien entendu, l'apprentissage peut se poursuivre dans un second temps avec le retour de l'utilisateur. Néanmoins, il y a nécessairement une phase d'apprentissage sans retour utilisateur.

Dans une autre réalisation de l'invention, chacune des touches du dispositif d'interface émotionnelle 70 a un ressenti tactile distinct les uns des autres et émet un stimulus olfactif et/ou visuel différent.

Avantageusement, le système 1 est configuré pour générer au moins un stimulus, et de préférence plusieurs stimuli. Les stimuli peuvent être de différentes natures comme des signaux olfactifs tels que des odeurs, des signaux visuels tels que des effets visuels sur un écran 32 ou par le biais d'un dispositif lumineux tel qu'une ou plusieurs ampoules ou une ou plusieurs lampes par exemple ou encore des signaux acoustiques tels que des sons. De préférence, les stimuli sont de nombres et de types différents.

### Fonctionnement du système 1

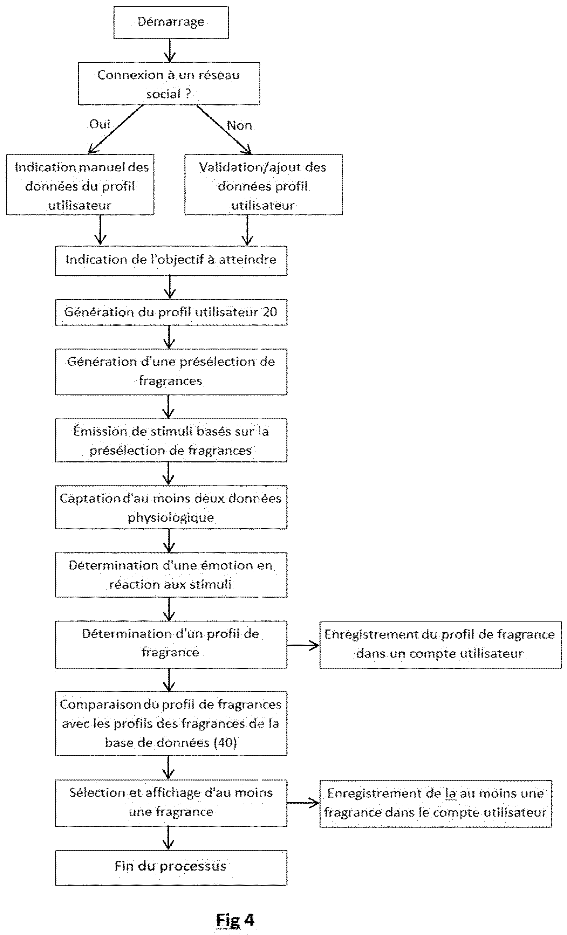

La figure 4 représente une réalisation du fonctionnement du système 1. Bien entendu, cette réalisation n'est pas limitative et des étapes peuvent être ajoutées et/ou inversées dans leurs ordres chronologiques.

Avantageusement, lors de l'utilisation du système 1, l'utilisateur va indiquer, dans un premier temps les données relatives à son objectif à atteindre. Cette indication peut se faire sous la forme d'un questionnaire, par exemple. Dans une réalisation de l'invention, l'objectif à atteindre est un état émotionnel spécifique. Pour ce faire, l'utilisateur peut utiliser les touches du dispositif d'interface émotionnelle 70.

Une fois son objectif indiqué, l'utilisateur renseigne son profil utilisateur 20. Une partie de ce profil utilisateur 20 peut être pré-remplie grâce à un appareil de prise de photographie ou vidéographie configuré pour réaliser une reconnaissance faciale et donner des informations relatives à une mesure ou une évaluation de la température ou de la sudation par exemple. Cette réalisation n'est pas visible dans la figure 4, mais parfaitement compatible avec la réalisation présentée dans cette dernière. Des moyens de reconnaissances faciales additionnelles comme une capture d'image dans le domaine des infrarouges peuvent être inclus dans le système 1. Ces moyens peuvent permettre d'améliorer la reconnaissance faciale. Dans un mode de réalisation, l'utilisateur est invité à confirmer les informations de son profil. Comme dit précédemment, le pré-remplissage des informations de l'utilisateur peut être réalisé par récupération de ses données sur au moins l'un des réseaux sociaux auquel est inscrit l'utilisateur.

Le système 1 va déterminer une présélection de parfum dans une base de données 40 comprenant une pluralité de parfums. Cette présélection est fonction du profil utilisateur 20 de l'utilisateur, de l'objectif de l'utilisateur et des profils des fragrances.

Le système 1 va ensuite émettre au moins un stimulus, et de préférence plusieurs stimuli. Ces stimuli sont, par exemple, des extraits des différents parfums présélectionnés, ou à tout le moins des senteurs proches.

Le but de ces stimuli est de générer un profil physiologique 31 de l'utilisateur en réponse à ces stimuli. Pour ce faire, le système 1 va capter au moins deux données physiologiques différentes, par exemple la température du corps de l'utilisateur ou encore son rythme cardiaque et son électroencéphalogramme. Les au moins deux données physiologiques captées par le capteur physiologique étant différentes de l'au moins une onde cérébrale. Avantageusement, la captation de ces éléments est en totale autonomie et sans retour de la part de l'utilisateur. Le but étant de déterminer un profil physiologique 31 de l'utilisateur sans son intervention. En effet, l'intervention de l'utilisateur dans la génération du profil physiologique 31 est une source de biais dans la génération dudit profil physiologique 31. En effet, si l'utilisateur est impliqué activement, c'est à dire par des choix conscients, lors de la sélection de la fragrance utilisateur, alors une dichotomie entre le besoin réel de l'utilisateur et sa volonté peut apparaitre. Cette dichotomie peut fausser le résultat de la sélection. Le profil physiologique 31 déterminé par l'invention dépend de l'état émotionnel de l'utilisateur, de préférence de son état émotionnel uniquement.

C'est pour ces raisons que le profil physiologique est généré suite auxdits plusieurs (c'est-à-dire au moins deux) stimuli. C'est-à-dire après et/ou pendant l'émission des stimuli. Ce sont lesdits stimuli qui permettent une réaction de l'utilisateur et ce sont ses réactions, captées par les capteurs physiologiques et le dispositif de capture d'au moins une onde cérébrale, qui permettent la génération d'un profil physiologique 31. La solution de l'état de la technique décrite dans le document WO2018/163361 ne prévoit pas cette solution. L'une des nombreuses différences entre la présente invention est ce document est que ce dernier se borne à sélectionner une fragrance en usant les données transmises sciemment par l'utilisateur et en modulant ces données par les données physiologiques récupérées par les capteurs avant l'émission d'un stimulus. Une fois la fragrance sélectionnée par le système, cette dernière est émise, possiblement avec d'autres stimuli afin de faire changer l'humeur de la personne. En cas d'échec, l'opération est recommencée. La présente invention, au contraire réalise plusieurs stimuli afin de parfaire le profil physiologique 31 avant la présentation de la fragrance utilisateur 50. De préférence, le présent système est configuré pour s'arrêter une fois que la fragrance utilisateur 50 ou groupe de fragrance utilisateur 50 est présenté à l'utilisateur. Dans le cadre du développement de la présente invention, il s'est avéré que cela permet d'obtenir des résultats bien différents et bien plus performants qu'avec la solution décrite dans l'état de la technique.

Pour ce faire, l'utilisateur doit porter des capteurs permettant de capter ces données physiologiques. Les capteurs peuvent être directement intégrés au système 1, ou être portés dès l'origine par l'utilisateur. Tel serait le cas de nombreux objets connectés portables, par exemple un téléphone intelligent, une montre connectée, un bracelet, etc. De manière alternative ou combinée, ils sont portés, associés ou reliés à une cabine 60. La cabine 60 peut être totalement ou partiellement fermée. Le contrôle de l'environnement de la cabine 60 est important. En effet, en contrôlant cet environnement, cela permet un affinage de la réponse aux stimuli, donc d'améliorer la création du profil physiologique 31. La cabine 60 peut se matérialiser par un espace dédié. On notera qu'une cabine 60 fermée permet une meilleure maitrise des stimuli.

Ainsi, la cabine 60 permet de positionner les diffuseurs de parfum à des endroits stratégiques et optimaux. Ainsi, la dose de parfum utilisée est réduite ce qui permet de limiter la saturation olfactive de l'utilisateur.

En effet, l'une des problématiques d'une cabine 60 est la saturation des odeurs lors de la génération de stimuli. En effet, la diffusion de plusieurs parfums dans un espace clos ou semiclos entraine une saturation rapide des capteurs olfactifs de l'utilisateur.

Pour limiter cet inconvénient, la cabine 60 comprend un système de diffusion des fragrances par micro-nébulisation ou par diffusion sèche.

De fait, les parfums sont stockés sous forme liquide prête à l'emploi.

Avantageusement les parfums diffusés comme stimuli ne sont pas dilués dans de l'alcool mais dans un autre solvant. Cette caractéristique avantageuse permet notamment de limiter l'effet de saturation des récepteurs olfactifs de l'utilisateur.

La cabine 60 permet aussi l'utilisation de diffuseur de parfum à sec. L'avantage d'une telle diffusion est qu'elle permet de limiter la saturation des récepteurs olfactifs de l'utilisateur. La diffusion à sec peut par exemple être réalisée par des diffuseurs comme le ScentysBox Interactive^{®}. Le document WO2018/163361 ne prévoit pas de telle diffusion et nécessite un solvant. Cette nécessité est notamment due au fait qu'il ne contrôle pas la localisation de la diffusion. Il faut donc une diffusion importante et puissante. Ce qui peut de plus être une gêne pour l'utilisateur.

Par exemple, le document WO2018/163361 divulgue des parfums stockés sous forme solide et comprenant un dissolvant sous forme d'alcool. Le document WO2018/163361 étant un dispositif portatif, il n'est pas possible d'optimiser la diffusion du parfum. De fait, la diffusion doit être puissante pour s'adapter à tous les environnements et simple à mettre en place (donc nécessite l'usage de parfums à base d'alcool). De plus, la fin des stimuli est contrôlée par l'utilisateur. Cela est obligatoire dans le cadre de ce document car il n'est pas possible de maitriser les conditions de l'environnement.

Ces problématiques sont résolues par l'utilisation d'une cabine 60 et d'une architecture de cabine spécifique permettant d'optimiser la diffusion du parfum. Cette optimisation permet de contrôler d'une part la dose délivrée, et d'autre part la composition de cette dose en enlevant les solvants alcoolisés. De fait, grâce à l'invention il est possible de délivrer plusieurs stimuli sans saturer les récepteurs olfactifs de l'utilisateur, et donc de permettre une génération de profil physiologique 31 plus précise.

La cabine 60 comprend l'ensemble des dispositifs de stimulus de l'utilisateur. Ainsi, la cabine 60 est équipée d'au moins et de préférence plusieurs écrans. Avantageusement, la cabine 60 comprend des écrans permettant à l'utilisateur d'avoir l'ensemble de son champ de vision occupé. L'avantage d'une telle disposition est de permettre une immersion et une relaxation totale de l'utilisateur. En effet, plus l'utilisateur sera détendu, plus ses paramètres physiologiques reflèteront la réponse aux stimuli et donc plus la sélection du parfum sera précise.

La cabine 60 comprend dans ses parois des diodes électroluminescentes (LED) permettant d'avoir un éclairage doux de l'ensemble de la cabine 60. Cet éclairage est configuré pour changer de couleurs en fonction de certains stimuli. Cela permet notamment d'accentuer le stimulus émis à l'attention de l'utilisateur.

Le système 1 peut aussi, si disponible, prendre en considération au moins un historique de certaines données physiologiques de l'utilisateur. Par exemple, l'objet connecté porté par l'utilisateur peut fournir au système 1 un historique des données qu'il a collectées. Ces éléments permettent de déterminer avec une plus grande précision le profil physiologique de l'utilisateur. Notamment, cela permet de comparer plus efficacement les variations dues aux stimuli avec l'état physiologique normal de l'utilisateur. Avantageusement, cette comparaison permet de détecter les émotions de l'utilisateur en réponse à un stimulus.

Dans une réalisation de l'invention, l'utilisateur est amené à décrire ses émotions grâce au dispositif d'interface émotionnelle 70. Cela permet de déterminer de possibles différences entre l'état physiologique de l'utilisateur et son ressenti émotionnel. Cela permet, d'une part, d'affiner le profil physiologique général, et d'autre part d'alimenter un algorithme d'intelligence artificielle des retours utilisateur de la sélection.

Dans une réalisation de l'invention, les étapes de génération du profil utilisateur 20 et du profil physiologique 31 sont réalisées au moins en partie ensemble. Par exemple, lorsque l'utilisateur renseigne les données du profil utilisateur 20, des stimuli sont émis par le système 1 et la réaction physiologique de l'utilisateur est captée à ce moment.

Une fois le profil utilisateur 20 et le profil physiologique 31 complétés, le système 1 calcule au moins une et, de préférence, la fragrance utilisateur et présente au moins une fragrance adéquate à l'objectif donné. La présentation de la fragrance utilisateur sélectionnée est faite sur un écran. De préférence, le système 1 présente une pluralité de fragrances adéquates afin de permettre à l'utilisateur de définir un profil de fragrance spécifique à l'utilisateur.

La sélection de l'au moins une fragrance adéquate est réalisée par au moins un algorithme mettant en place une intelligence artificielle 45. Cette intelligence artificielle 45 peut ainsi apprendre le comportement d'au moins un utilisateur afin de sélectionner les fragrances adéquates. Cette sélection est notamment réalisée en fonction du profil utilisateur 20 couplé au profil physiologique 31 avec les différentes fragrances.

L'ensemble des données récoltées lors de l'utilisation du système 1 est enregistré dans une base de données système 1, de préférence de manière anonyme, afin d'alimenter un algorithme d'apprentissage. Ainsi, en fonction du nombre d'utilisateurs, des améliorations de la présélection et de la sélection du parfum seront possibles. Le système 1 détectera plus facilement les émotions réelles des utilisateurs. Le système 1 associera avec plus de précision les profils de chaque utilisateur avec les profils des fragrances.

La constitution d'une base de données permet l'amélioration de l'intelligence artificielle 45 permettant la sélection de la fragrance personnalisée. Notamment, la base de données comprend l'association pour chacune des fragrances du profil physiologique 31 à atteindre pour un objectif donné. Autrement dit, pour chacune des fragrances en fonction de l'objectif à atteindre, la base de données lui associe des réponses physiologiques et cérébrales en fonction des différents stimuli. Ainsi, le système peut comparer les données physiologiques et cérébrales émises par l'utilisateur en réponse aux stimuli afin de déterminer si la fragrance est optimale ou si de nouveaux stimuli sont nécessaires afin d'affiner la sélection de la fragrance. Le profil physiologique 31 comprend en outre un historique de réponses de l'utilisateur aux stimuli générés et destinés à être reçus par l'utilisateur, le système étant configuré de sorte que ledit historique soit généré en mettant en œuvre les étapes suivantes :
- Identification, en fonction des objectifs à atteindre et de données personnelles, d'un profil cible préenregistré dans la base de données,
- Au moins un cycle, et de préférence au moins deux cycles, chaque cycle comprenant les étapes successives suivantes, certains au moins de ces étapes étant effectuées par un système informatique de traitement de données:
   a. Génération d'au moins un stimulus additionnel,
   b. Captation d'au moins une donnée physiologique et d'un signal cérébral issus d'une onde cérébrale de l'utilisateur, la donnée physiologique et l'onde cérébrale étant générées par l'utilisateur en réponse audit au moins un stimulus additionnel,
   c. Actualisation du profil physiologique en fonction des données physiologiques et de l'onde cérébrale générée par l'utilisateur en réponse audit au moins un stimulus additionnel,
   d. Comparaison du profil physiologique actualisé avec des profils préenregistrés dans la base de données,
   e. Si le profil physiologique actualisé:
      - correspond à un profil préenregistré dans la base de données, alors génération de l'historique sur la base des stimuli additionnels de tous les cycles,
      - ne correspond à aucun profil préenregistré dans la base de données, alors répétition des étapes a à d avec des stimuli présentant des paramètres différents d'un cycle à l'autre.

Ainsi, des stimuli sont fournis à l'utilisateur jusqu'à ce que le profil physiologique corresponde à un profil préenregistré. Le profil physiologique prend alors en compte l'ensemble des stimuli qui ont permis d'amener l'utilisateur jusqu'au profil cible.

Ainsi, cette prise en compte de l'historique dans le profil physiologique permet de sélectionner de manière rapide, automatique la fragrance qui convient à l'utilisateur pour l'amener dans l'état émotionnel qu'il souhaite et qui lui correspond. Par ailleurs, cette sélection est beaucoup plus précise que dans les solutions de l'état de la technique car basée sur les réactions physiologiques de l'utilisateur, de préférence en temps réel, et non pas sur son état conscient
Ces étapes d'apprentissage permettent une actualisation du profil physiologique personnalisé en fonction des réactions de l'utilisateur aux stimuli, et ainsi permet une amélioration de la détermination de la fragrance utilisateur 50 à sélectionner.

De plus, cette actualisation du profil physiologique 31 peut permettre par exemple la modulation de la délivrance des stimuli en fonction des données physiologiques de l'utilisateur et donc d'éviter des effets indésirables tels que la génération de stress lors de l'émission des stimuli à destinations de l'utilisateur. Cela permet aussi un contrôle de l'ensemble des paramètres des stimuli lors de la génération du profil physiologique 31.

Avantageusement, la combinaison du profil utilisateur 20 et du profil physiologique 31 permet la création du profil de fragrance utilisateur. Ainsi, il est possible d'associer à chaque utilisateur un ensemble de plusieurs fragrances. En fonction des objectifs à atteindre par l'utilisateur, ce dernier pourra choisir le parfum optimal dans une liste réduite de fragrances.

Le système 1 peut comprendre la création de comptes personnels d'un utilisateur. Ainsi, chaque utilisateur peut retrouver l'ensemble de son profil utilisateur 20, de son profil physiologique 31 et plus généralement son profil de fragrance utilisateur et l'ensemble des parfums associés. Cette solution a notamment l'avantage de mettre à jour cette sélection en fonction des nouvelles fragrances ajoutées dans la base de données 40.

Au final, le système 1 peut présenter une ou plusieurs fragrances utilisateur 50 en fonction des profils utilisateur 20 et physiologique 31 de l'utilisateur.

Dans une réalisation de l'invention, le système 1 prend la forme d'une cabine 60 dans laquelle l'utilisateur est isolé lors de l'utilisation du système 1.

L'invention n'est pas limitée aux modes de réalisation précédemment décrits et s'étend à tous les modes de réalisation couverts par les revendications.

### Référence numérique

1. Système
10. Interface utilisateur
20. Profil utilisateur
30. Capteur physiologique
31. Profil physiologique
32. Écran
33. Diffuseur d'odeur
34. Enceinte
35. Capteur optique
36. Casque neuronal
40. Base de données
45. Intelligence artificielle
50. Fragrance utilisateur
60. Cabine
70. Dispositif d'interface émotionnelle

## Revendications

1. Système (1) d'aide à la sélection par un utilisateur d'une fragrance dans une base de données (40) comportant une pluralité de fragrances, **caractérisé en ce que** le système comprend :
a) une interface utilisateur (10) configurée pour recevoir et transmettre au système (1) au moins une donnée relative à un objectif à atteindre et un profil utilisateur (20) de l'utilisateur, la donnée relative à un objectif à atteindre et le profil utilisateur (20) étant fonction de données personnelles fournies directement ou indirectement par l'utilisateur,
b) un dispositif configuré pour générer plusieurs stimuli destinés à être reçus par l'utilisateur, lesdits stimuli étant des stimuli olfactifs, visuels et/ou sonores,
c) au moins deux capteurs physiologiques (30) configurés pour capter au moins deux données physiologiques différentes de l'utilisateur en réponse auxdits stimuli,
d) un dispositif configuré pour capter au moins une onde cérébrale générée par l'utilisateur en réponse auxdits plusieurs stimuli et pour générer au moins un signal cérébral en fonction de cette onde cérébrale, le signal cérébral étant différent des au moins deux données physiologiques (30)
e) le système (1) étant configuré pour générer un profil physiologique (31) à partir au moins des au moins deux données physiologiques et du signal cérébral,
et pour sélectionner au moins une fragrance utilisateur (50) dans la base de données (40) en fonction dudit profil physiologique (31) généré à partir des au moins deux données physiologiques et du signal cérébral, du profil utilisateur (20) et de la donnée relative à un objectif à atteindre.

2. Système (1) selon la revendication précédente dans lequel le profil utilisateur (20) comprend au moins une donnée prise parmi l'âge, le sexe, le genre, le trait de caractère ou l'origine ethnique de l'utilisateur et/ou les au moins deux données physiologiques différentes de l'onde cérébrale et captées par le capteur physiologique sont prises parmi la température, la tension artérielle, la sudation, l'émotion, le rythme respiratoire, la conductivité de la peau, la composition de la salive, le mouvement des yeux, la dilatation de la pupille ou le rythme cardiaque de l'utilisateur, préférentiellement le système comprenant au moins deux et de préférence au moins trois capteurs physiologiques (30) configuré pour capter au moins trois données physiologiques différentes de l'utilisateur en réponse audit au moins un stimulus.

3. Système (1) selon l'une quelconque des revendications précédentes dans lequel chacune des fragrances de la base de données (40) comprend un profil de fragrance distinct des autres fragrances,
a) le système (1) est, en outre, configuré pour déterminer un profil de fragrance utilisateur en fonction du profil physiologique (31), du profil utilisateur (20) et de la donnée relative à un objectif à atteindre,
et comprenant un algorithme configuré pour :
a) comparer le profil de fragrance utilisateur et le profil de chacune des fragrances de la base de données (40) comportant une pluralité de fragrances et
b) sélectionner l'au moins une fragrance utilisateur (50) en fonction de ladite comparaison.

4. Système (1) selon l'une quelconque des revendications précédentes comprenant un dispositif d'interface émotionnelle (70) configuré pour réaliser une interface entre l'utilisateur et le système (1) et permettant à l'utilisateur d'indiquer son état émotionnel avant l'émission des au moins deux stimuli et préférentiellement permettant à l'utilisateur d'actualiser le profil physiologique (31) suite aux stimuli et dans lequel la sélection de l'au moins une fragrance utilisateur (50) est fonction du profil physiologique (31) actualisé, du profil utilisateur (20), de la donnée relative à un objectif à atteindre et de l'état émotionnel de l'utilisateur, préférentiellement, le dispositif d'interface émotionnelle (70) est configuré pour recevoir et transmettre au système (1) au moins une donnée relative à un état émotionnel de l'utilisateur défini avant émission d'un stimulus et qualifié d'état émotionnel pré-stimulus, préférentiellement le système étant configuré pour comparer l'au moins une donnée physiologique de l'utilisateur captée avant ledit au moins l'un des stimulus et l'au moins une donnée physiologique de l'utilisateur captée après ledit au moins un stimulus afin de vérifier l'état émotionnel de l'utilisateur.

5. Système (1) selon la revendication précédente dans lequel le dispositif d'interface émotionnelle (70) comprend une pluralité de touches, et dans lequel chaque touche est configurée pour indiquer un état émotionnel de l'utilisateur, de préférence par la génération d'un stimulus tactile et/ou visuel différent pour chacune des touches, et/ou chacune des touches du dispositif d'interface émotionnelle (70) comprend un revêtement tactile différent associé à un état émotionnel spécifique, et/ou chacune des touches du dispositif d'interface émotionnelle (70) comprend une couleur différente associée un état émotionnel spécifique et/ou chaque touche comprend un dispositif permettant de générer au moins un stimulus pris parmi le stimulus ou un stimulus additionnel différent d'autre stimulus auprès de l'utilisateur permettant audit utilisateur d'indiquer son état émotionnel.

6. Système (1) selon l'une des revendications précédentes configuré pour, de préférence par l'intermédiaire d'un dispositif de reconnaissance faciale du système tel que par exemple une caméra ou un appareil photographique, déterminer en fonction de la reconnaissance faciale de l'utilisateur au moins l'un parmi l'âge de l'utilisateur, le sexe de l'utilisateur, le genre de l'utilisateur, l'émotion de l'utilisateur, l'origine ethnique, la température de l'utilisateur, la sudation de l'utilisateur.

7. Système (1) selon l'une quelconque des revendications précédentes dans lequel le profil physiologique est généré sans que l'utilisateur ne donne sciemment d'instructions en réponse auxdits au moins deux stimuli.

8. Système selon l'une quelconque des revendications précédentes dans lequel la fragrance utilisateur (50) est sélectionnée sans que l'utilisateur ne donne sciemment d'instructions en réponse auxdits au moins deux stimuli.

9. Système (1) selon l'une quelconque des revendications précédentes dans lequel la génération par le système (1) du profil physiologique (31) à partir de l'au moins une donnée physiologique, et la sélection par ledit système (1) d'au moins une fragrance utilisateur (50) dans la base de données (40) en fonction du profil physiologique (31), du profil utilisateur (20) et de la donnée relative à un objectif à atteindre est réalisé par une série d'algorithmes formant une intelligence artificielle (45).

10. Système (1) selon l'une quelconque des revendications précédentes dans lequel le profil physiologique (31) comprend en outre un historique de réponses de l'utilisateur aux stimuli générés et destinés à être reçus par l'utilisateur, le système étant configuré de sorte que ledit historique soit généré en mettant en œuvre les étapes suivantes :
- Identification, en fonction des objectifs à atteindre de données personnelles, d'un profil cible préenregistré dans la base de données,
- Au moins un cycle, et de préférence au moins deux cycles, chaque cycle comprenant les étapes successives suivantes, certains au moins de ces étapes étant effectuées par un système informatique de traitement de données:
a) Génération d'au moins un stimulus additionnel,
b) Captation d'au moins une donnée physiologique et d'un signal cérébral issus d'une onde cérébrale de l'utilisateur, la donnée physiologique et l'onde cérébrale étant générées par l'utilisateur en réponse audit au moins un stimulus additionnel,
c) Actualisation du profil physiologique en fonction des données physiologiques et de l'onde cérébrale générée par l'utilisateur en réponse audit au moins un stimulus additionnel,
d) Comparaison du profil physiologique actualisé avec des profils préenregistrés dans la base de données,
e) Si le profil physiologique actualisé:
- correspond à un profil préenregistré dans la base de données, alors génération de l'historique sur la base des stimuli additionnels de tous les cycles,
- ne correspond à aucun profil préenregistré dans la base de données, alors répétition des étapes a à d avec des stimuli présentant des paramètres différents d'un cycle à l'autre.

11. Procédé d'utilisation du système (1) selon l'une quelconque des revendications précédentes pour aider un utilisateur à la sélection d'une fragrance dans une base de données (40) comportant une pluralité de fragrances comportant chacune un profil de fragrance, **caractérisé en ce que** le procédé d'aide à la sélection d'une fragrance comprend les étapes suivantes mises en œuvre par un système informatique à l'aide d'au moins un microprocesseur :
a) Génération d'une donnée relative à un objectif à atteindre et d'un profil utilisateur (20) de l'utilisateur, la donnée relative à un objectif à atteindre et le profil utilisateur (20) étant fonction de données personnelles fournies directement ou indirectement par l'utilisateur;
b) Génération d'une présélection de fragrances basée notamment sur la donnée relative à un objectif à atteindre, le profil utilisateur (20) et sur des caractéristiques chimiques des fragrances stockées dans la base de données (40),
c) Génération d'au moins deux, stimuli destinés à être reçus par l'utilisateur, lesdits au moins deux stimuli sont pris parmi des stimuli olfactifs, visuels et/ou sonores,
d) Génération d'un profil physiologique (31) basé au moins sur :
i. au moins un signal cérébral, le signal cérébral étant basé sur au moins une onde cérébrale générée par l'utilisateur en réponse auxdits au moins deux stimuli;
ii. au moins deux données physiologiques de l'utilisateur, différentes de l'au moins une onde cérébrale, lesdites au moins deux données physiologiques de l'utilisateur étant captées en réponse à la génération et la captation d'au moins deux stimuli;
e) Détermination par un algorithme d'un profil de fragrance utilisateur en fonction du profil physiologique (31), du profil utilisateur (20) et de la donnée relative à un objectif à atteindre de l'utilisateur;
f) Comparaison du profil de fragrance utilisateur déterminé par l'algorithme avec les profils des fragrances de la base de données (40) et sélection d'au moins une fragrance utilisateur (50) ;
g) Affichage de l'au moins une fragrance utilisateur (50) sélectionnée, de préférence à l'aide d'un écran.

12. Procédé d'utilisation selon la revendication précédente dans lequel les stimuli présentent plusieurs paramètres, de préférence pris parmi une nature de stimulus, une durée, une intensité, lesdits paramètres étant déterminés automatiquement en fonction au moins desdites données personnelles fournies directement ou indirectement par l'utilisateur et/ou par les données physiologiques et l'au moins un signal cérébral capté respectivement par les au moins deux capteurs physiologiques (30) et le dispositif de capture de l'au moins une onde cérébrale, préférentiellement le profil physiologique (31) comprend en outre un historique de réponses de l'utilisateur aux stimuli générés et destinés à être reçus par l'utilisateur .

13. Procédé selon la revendication précédente dans lequel ledit historique est généré en effectuant les étapes suivantes :
- Identification, en fonction des objectifs à atteindre et de données personnelles, d'un profil cible préenregistré dans la base de données,
- Au moins un cycle, et de préférence au moins deux cycles, de préférence au moins cinq cycles, chaque cycle comprenant les étapes successives suivantes, certains au moins de ces étapes étant effectuées par un système informatique de traitement de données:
a) Génération d'au moins un stimulus additionnel,
b) Captation d'au moins une donnée physiologique et d'un signal cérébral issus d'une onde cérébrale de l'utilisateur, la donnée physiologique et l'onde cérébrale étant générées par l'utilisateur en réponse audit au moins un stimulus additionnel,
c) Actualisation du profil physiologique en fonction des données physiologiques et de l'onde cérébrale générée par l'utilisateur en réponse audit au moins un stimulus additionnel,
d) Comparaison du profil physiologique actualisé avec des profils préenregistrés dans la base de données,
e) Si le profil physiologique actualisé:
- correspond à un profil préenregistré dans la base de données, alors génération de l'historique sur la base des stimuli additionnels de tous les cycles,
- ne correspond à aucun profil préenregistré dans la base de données, alors répétition des étapes a à d avec des stimuli présentant des paramètres différents d'un cycle à l'autre, préférentiellement, l'utilisateur n'interagit pas sciemment avec le système lors de la génération du profil physiologique (31).

14. Cabine (60) comprenant un système (1) selon l'une des revendications 1 à 10 comprenant un casque neuronal (36) configuré pour capter l'au moins une onde cérébrale, une interface utilisateur (10), un écran (32) et au moins le capteur physiologique (30) configuré pour capteur les au moins deux données physiologiques différentes et différentes de l'au moins une onde cérébrale captée par le casque neuronal (36).

15. Cabine (60) selon la revendication précédente comprenant un système de diffusion d'un parfum par diffusion sèche.

## Patentansprüche

1. System (1) zur Unterstützung eines Benutzers bei der Auswahl eines Dufts aus einer Datenbank (40), die eine Vielzahl von Düften aufweist, **dadurch gekennzeichnet, dass** das System Folgendes umfasst:
a) eine Benutzerschnittstelle (10), die so eingerichtet ist, dass sie mindestens ein Datenelement in Bezug auf ein zu erreichendes Ziel und ein Benutzerprofil (20) des Benutzers empfängt und an das System (1) überträgt, wobei das Datenelement in Bezug auf ein zu erreichendes Ziel und das Benutzerprofil (20) von personenbezogenen Daten abhängen, die direkt oder indirekt von dem Benutzer bereitgestellt werden,
b) eine Vorrichtung, die so eingerichtet ist, dass sie mehrere Stimuli erzeugt, die von dem Benutzer empfangen werden sollen, wobei die Reize olfaktorische, visuelle und/oder akustische Reize sind,
c) mindestens zwei physiologische Sensoren (30), die so eingerichtet sind, dass sie als Reaktion auf die Stimuli mindestens zwei verschiedene physiologische Daten des Benutzers erfassen,
d) eine Vorrichtung, die so eingerichtet ist, dass sie mindestens eine von dem Benutzer als Reaktion auf die mehreren Stimuli erzeugte Gehirnwelle empfängt und mindestens ein Gehirnsignal in Abhängigkeit von dieser Gehirnwelle erzeugt, wobei sich das Gehirnsignal von den mindestens zwei physiologischen Daten (30) unterscheidet
e) wobei das System (1) so eingerichtet ist, dass es anhand der mindestens zwei physiologischen Daten und des Gehirnsignals ein physiologisches Profil (31) erzeugt,
und zum Auswählen mindestens eines Benutzerdufts (50) aus der Datenbank (40) in Abhängigkeit von dem physiologischen Profil (31), das anhand der mindestens zwei physiologischen Daten und des Gehirnsignals erzeugt wurde, dem Benutzerprofil (20) und dem Datenelement in Bezug auf ein zu erreichendes Ziel erzeugt wird.

2. System (1) nach dem vorhergehenden Anspruch, wobei das Benutzerprofil (20) mindestens eine Datenelement umfasst, das aus Alter, Geschlecht, Typ, Charakterzügen oder ethnischer Herkunft des Benutzers ausgewählt ist, und/oder die mindestens zwei von der Gehirnwelle unterschiedlichen und von dem physiologischen Sensor erfassten physiologischen Daten aus Temperatur, Blutdruck, Schweißabsonderung, Emotionen, Atemrhythmus, Leitfähigkeit der Haut, Speichelzusammensetzung, Augenbewegungen, Erweiterung der Pupillen oder Herzrhythmus des Benutzers ausgewählt sind, wobei das System vorzugsweise mindestens zwei und bevorzugt mindestens drei physiologische Sensoren (30) umfasst, die so eingerichtet sind, dass sie als Reaktion auf den mindestens einen Stimulus mindestens drei verschiedene physiologische Daten des Benutzers erfassen.

3. System (1) nach einem der vorhergehenden Ansprüche, wobei jeder der Düfte aus der Datenbank (40) ein von den anderen Düften verschiedenes Duftprofil umfasst,
a) wobei das System (1) ferner so eingerichtet ist, dass es ein Benutzerduftprofil in Abhängigkeit von dem physiologischen Profil (31), von dem Benutzerprofil (20) und dem Datenelement in Bezug auf ein zu erreichendes Ziel bestimmt,
und umfassend einen Algorithmus, der für Folgendes eingerichtet ist:
a) Vergleichen des Benutzerduftprofils und des Profils jedes der Düfte aus der Datenbank (40), die eine Vielzahl von Düften aufweist, und
b) Auswählen des mindestens einen Benutzerdufts (50) in Abhängigkeit von dem Vergleich.

4. System (1) nach einem der vorhergehenden Ansprüche, das eine emotionale Schnittstellenvorrichtung (70) umfasst, die so eingerichtet ist, dass sie eine Schnittstelle zwischen dem Benutzer und dem System (1) herstellt und es dem Benutzer ermöglicht, seinen emotionalen Zustand vor der Ausgabe der mindestens zwei Stimuli anzugeben, und es vorzugsweise dem Benutzer ermöglicht, das physiologische Profil (31) infolge der Stimuli zu aktualisieren, und wobei die Auswahl des mindestens einen Benutzerdufts (50) abhängig von dem aktualisierten physiologischen Profil (31), von dem Benutzerprofil (20), von dem Datenelement in Bezug auf ein zu erreichendes Ziel und von dem emotionalen Zustand des Benutzers ist, wobei die emotionale Schnittstellenvorrichtung (70) vorzugsweise so eingerichtet ist, dass sie mindestens ein Datenelement in Bezug auf einen emotionalen Zustand des Benutzers empfängt und an das System (1) überträgt, der vor der Ausgabe eines Stimulus definiert und als emotionaler Zustand vor dem Stimulus bezeichnet wird, wobei das System vorzugsweise so eingerichtet ist, dass es das mindestens eine vor dem mindestens einen der Stimuli erfasste physiologische Datenelement des Benutzers und das mindestens eine nach dem mindestens einen Stimulus erfasste physiologische Datenelement des Benutzers vergleicht, um den emotionalen Zustand des Benutzers zu überprüfen.

5. System (1) nach dem vorhergehenden Anspruch, wobei die emotionale Schnittstellenvorrichtung (70) eine Vielzahl von Tasten umfasst, und wobei jede Taste so eingerichtet ist, dass sie einen emotionalen Zustand des Benutzers anzeigt, bevorzugt durch Erzeugung eines unterschiedlichen taktilen und/oder visuellen Stimulus für jede der Tasten, und/oder jede der Tasten der emotionalen Schnittstellenvorrichtung (70) eine unterschiedliche taktile Beschichtung umfasst, die einem bestimmten emotionalen Zustand zugeordnet ist, und/oder jede der Tasten der emotionalen Schnittstellenvorrichtung (70) eine andere Farbe umfasst, die einem bestimmten emotionalen Zustand zugeordnet ist, und/oder jede Taste eine Vorrichtung umfasst, die es ermöglicht, mindestens einen Stimulus aus dem Stimulus oder einen zusätzlichen Stimulus, der sich von einem anderen Stimulus bei dem Benutzer unterscheidet, zu erzeugen, sodass der Benutzer seinen emotionalen Zustand anzeigen kann.

6. System (1) nach einem der vorhergehenden Ansprüche, das so eingerichtet ist, dass es, bevorzugt über eine Gesichtserkennungsvorrichtung des Systems wie beispielsweise eine Kamera oder einen Fotoapparat, in Abhängigkeit von der Gesichtserkennung des Benutzers mindestens eines aus Alter des Benutzers, Geschlecht des Benutzers, Typ des Benutzers, Emotion des Benutzers, ethnischer Herkunft, Temperatur des Benutzers und Schweißabsonderung des Benutzers bestimmt.

7. System (1) nach einem der vorhergehenden Ansprüche, wobei das physiologische Profil erzeugt wird, ohne dass der Benutzer bewusst Anweisungen als Reaktion auf die mindestens zwei Stimuli gibt.

8. System nach einem der vorhergehenden Ansprüche, wobei der Benutzerduft (50) ausgewählt wird, ohne dass der Benutzer bewusst Anweisungen als Reaktion auf die mindestens zwei Stimuli gibt.

9. System (1) nach einem der vorhergehenden Ansprüche, wobei das Erzeugen des physiologischen Profils (31) anhand des mindestens einen physiologischen Datenelements durch das System (1) und die Auswahl von mindestens einem Benutzerduft (50) aus der Datenbank (40) durch das System (1) in Abhängigkeit von dem physiologischen Profil (31), dem Benutzerprofil (20) und dem Datenelement in Bezug auf ein zu erreichendes Ziel durch eine Reihe von Algorithmen, die eine künstliche Intelligenz (45) bilden, erfolgt.

10. System (1) nach einem der vorhergehenden Ansprüche, wobei das physiologische Profil (31) ferner eine Historie der Reaktionen des Benutzers auf die Stimuli, die erzeugt wurden und von dem Benutzer empfangen werden sollten, umfasst, wobei das System so eingerichtet ist, dass die Historie durch das Durchführen der folgenden Schritte erzeugt wird:
- Identifizieren eines in der Datenbank vorab gespeicherten Zielprofils in Abhängigkeit von den zu erreichenden Zielen der personenbezogenen Daten,
- mindestens einen Zyklus, und bevorzugt mindestens zwei Zyklen, wobei jeder Zyklus die folgenden aufeinanderfolgenden Schritte umfasst, wobei mindestens einige dieser Schritte von einem Datenverarbeitungssystem durchgeführt werden:
a) Erzeugen mindestens eines zusätzlichen Stimulus,
b) Erfassen mindestens eines physiologischen Datenelements und eines Gehirnsignals, das von einer Gehirnwelle des Benutzers stammt, wobei das physiologische Datenelement und die Gehirnwelle von dem Benutzer als Reaktion auf den mindestens einen zusätzlichen Stimulus erzeugt werden,
c) Aktualisieren des physiologischen Profils in Abhängigkeit von den physiologischen Daten und der von dem Benutzer als Reaktion auf den mindestens einen zusätzlichen Stimulus erzeugten Gehirnwelle,
d) Vergleichen des aktualisierten physiologischen Profils mit den in der Datenbank vorab gespeicherten Profilen,
e) wenn das aktualisierte physiologische Profil:
- einem in der Datenbank vorab gespeicherten Profil entspricht, dann Erzeugen der Historie auf der Grundlage der zusätzlichen Stimuli aller Zyklen,
- keinem in der Datenbank vorab gespeicherten Profil entspricht, dann Wiederholen der Schritte a bis d mit Stimuli, die von Zyklus zu Zyklus unterschiedliche Parameter besitzen.

11. Verfahren zur Verwendung des Systems (1) nach einem der vorhergehenden Ansprüche, um einen Benutzer bei der Auswahl eines Dufts aus einer Datenbank (40) zu unterstützen, die eine Vielzahl von Düften aufweist, die jeweils ein Duftprofil aufweisen, **dadurch gekennzeichnet, dass** das Verfahren zur Unterstützung bei der Auswahl eines Dufts die folgenden Schritte umfasst, die von einem Computersystem mithilfe von mindestens einem Mikroprozessor durchgeführt werden:
a) Erzeugen eines Datenelements in Bezug auf ein zu erreichendes Ziel und eines Benutzerprofils (20) des Benutzers, wobei das Datenelement in Bezug auf ein zu erreichendes Ziel und das Benutzerprofil (20) von personenbezogenen Daten abhängen, die direkt oder indirekt von dem Benutzer bereitgestellt werden;
b) Erzeugen einer Duftvorauswahl, die insbesondere auf dem Datenelement in Bezug auf ein zu erreichendes Ziel, dem Benutzerprofil (20) und den chemischen Eigenschaften der in der Datenbank (40) gespeicherten Düfte basiert,
c) Erzeugen von mindestens zwei Stimuli, die von dem Benutzer empfangen werden sollen, wobei die mindestens zwei Stimuli aus olfaktorischen, visuellen und/oder akustischen Stimuli aufgewählt werden,
d) Erzeugen eines physiologischen Profils (31) basierend auf mindestens einem von Folgendem:
i. mindestens einem Gehirnsignal, wobei das Gehirnsignal auf mindestens einer von dem Benutzer als Reaktion auf die mindestens zwei Stimuli erzeugten Gehirnwelle basiert;
ii. mindestens zwei physiologische Daten des Benutzers, die sich von der mindestens einen Gehirnwelle unterscheiden, wobei die mindestens zwei physiologischen Daten des Benutzers als Reaktion auf das Erzeugen und das Erfassen von mindestens zwei Stimuli erfasst werden;
e) Bestimmen eines Benutzerduftprofils durch einen Algorithmus in Abhängigkeit von dem physiologischen Profil (31), dem Benutzerprofil (20) und dem Datenelement in Bezug auf ein zu erreichendes Ziel des Benutzers;
f) Vergleichen des von dem Algorithmus bestimmten Benutzerduftprofils mit den Duftprofilen aus der Datenbank (40) und Auswählen mindestens eines Benutzerdufts (50);
g) Anzeigen des mindestens einen ausgewählten Benutzerdufts (50), bevorzugt mit Hilfe eines Bildschirms.

12. Verwendungsverfahren nach dem vorhergehenden Anspruch, wobei die Stimuli mehrere Parameter besitzen, die bevorzugt aus einer Stimulusart, einer Dauer und einer Intensität ausgewählt sind, wobei die Parameter automatisch in Abhängigkeit von mindestens den von dem Benutzer direkt oder indirekt bereitgestellten personenbezogenen Daten und/oder von den physiologischen Daten und dem mindestens einen von den mindestens zwei physiologischen Sensoren (30) und der Vorrichtung zur Erfassung der mindestens einen Gehirnwelle erfassten Gehirnsignal bestimmt werden, wobei das physiologische Profil (31) vorzugsweise ferner eine Historie der Reaktionen des Benutzers auf die Stimuli, die erzeugt wurden und von dem Benutzer empfangen werden sollten, umfasst.

13. Verfahren nach dem vorhergehenden Anspruch, wobei die Historie durch das Durchführen der folgenden Schritte erzeugt wird:
- Identifizieren eines in der Datenbank vorab gespeicherten Zielprofils in Abhängigkeit von den zu erreichenden Zielen und den personenbezogenen Daten,
- mindestens einen Zyklus, und bevorzugt mindestens zwei Zyklen, bevorzugt mindestens fünf Zyklen, wobei jeder Zyklus die folgenden aufeinanderfolgenden Schritte umfasst, wobei mindestens einige dieser Schritte von einem Datenverarbeitungssystem durchgeführt werden:
a) Erzeugen mindestens eines zusätzlichen Stimulus,
b) Erfassen mindestens eines physiologischen Datenelements und eines Gehirnsignals, das von einer Gehirnwelle des Benutzers stammt, wobei das physiologische Datenelement und die Gehirnwelle von dem Benutzer als Reaktion auf den mindestens einen zusätzlichen Stimulus erzeugt werden,
c) Aktualisieren des physiologischen Profils in Abhängigkeit von den physiologischen Daten und der von dem Benutzer als Reaktion auf den mindestens einen zusätzlichen Stimulus erzeugten Gehirnwelle,
d) Vergleichen des aktualisierten physiologischen Profils mit den in der Datenbank vorab gespeicherten Profilen,
e) wenn das aktualisierte physiologische Profil:
- einem in der Datenbank vorab gespeicherten Profil entspricht, dann Erzeugen der Historie auf der Grundlage der zusätzlichen Stimuli aller Zyklen,
- keinem in der Datenbank vorgespeicherten Profil entspricht, dann Wiederholen der Schritte a bis d mit Stimuli, die von Zyklus zu Zyklus unterschiedliche Parameter besitzen, wobei der Benutzer vorzugsweise bei der Erzeugung des physiologischen Profils (31) nicht bewusst mit dem System interagiert.

14. Kabine (60), die ein System (1) nach einem der Ansprüche 1 bis 10 umfasst, das einen neuronalen Helm (36), der so eingerichtet ist, dass er die mindestens eine Gehirnwelle erfasst, eine Benutzerschnittstelle (10), einen Bildschirm (32) und den mindestens physiologischen Sensor (30) umfasst, der so eingerichtet ist, dass er die mindestens zwei unterschiedlichen Daten erfasst, die sich von der mindestens einen von dem neuronalen Helm (36) erfassten Gehirnwelle unterscheiden.

15. Kabine (60) nach dem vorhergehenden Anspruch, die ein System zur Diffusion eines Parfüms durch Trockendiffusion umfasst.

## Claims

1. A system (1) for assisting a user in selecting a fragrance from a database (40) containing a plurality of fragrances, **characterised in that** the system comprises:
a) a user interface (10) configured to receive and transmit to the system (1) at least one piece of data relating to an objective to be achieved and a user profile (20) of the user, the piece of data relating to the objective to be achieved and the user profile (20) being based on personal data provided directly or indirectly by the user,
b) a device configured to generate several stimuli to be received by the user, said stimuli being olfactory, visual and/or sound stimuli,
c) at least two physiological sensors (30) configured to sense at least two different pieces of physiological data of the user in response to said stimuli,
d) a device configured to sense at least one brain wave generated by the user in response to said several stimuli and to generate at least one brain signal based on this brain wave, the brain signal being different from the at least two pieces of physiological data (30)
e) the system (1) being configured to generate a physiological profile (31) at least from the at least two physiological pieces of data and the brain signal,
and to select at least one user fragrance (50) from the database (40) based on the physiological profile (31) generated from the at least two physiological pieces of data and the brain signal, the user profile (20), and the piece of data relating to an objective to be achieved.

2. The system (1) according to the preceding claim, wherein the user profile (20) comprises at least one piece of data selected from age, sex, gender, character trait, or ethnic origin of the user and/or the at least two different physiological pieces of data of the brain wave sensed by the physiological sensor are taken from temperature, blood pressure, sweating, emotion, respiratory rate, skin conductivity, saliva composition, eye movement, pupil dilation, or heart rate of the user; preferably, the system comprises at least two, and preferably at least three physiological sensors (30) configured to sense at least three different physiological pieces of data of the user in response to said at least one stimulus.

3. The system (1) according to any one of the preceding claims, in which each of the fragrances in the database (40) has a fragrance profile distinct from the other fragrances,
a) the system (1) is further configured to determine a user fragrance profile depending on the physiological profile (31), the user profile (20), and the piece of data relating to an objective to be achieved,
and comprising an algorithm configured to:
a) compare the user fragrance profile with the profile of each of the fragrances in the database (40), including a plurality of fragrances and
b) selecting the at least one user fragrance (50) based on said comparison.

4. The system (1) according to any one of the preceding claims, comprising an emotional interface device (70) configured to make an interface between the user and the system (1), allowing the user to indicate their emotional state prior to emitting the at least two stimuli, and preferably enabling the user to update the physiological profile (31) as a result of the stimuli, and wherein the selection of at least one user fragrance (50) is based on the physiological profile (31) updated, the user profile (20), the piece of data relating to a objective to be achieved, and the user's emotional state, preferably, the emotional interface device (70) is configured to receive and transmit to the system (1) at least one piece of data relating to the user's emotional state defined prior to emitting a stimulus and referred to as the pre-stimulus emotional state, preferably, the system is configured to compare the at least one physiological piece of data of the user sensed before said at least one of the stimuli with the at least one physiological piece of data sensed said at least one stimulus in order to verify the user's emotional state.

5. The system (1) according to the preceding claim, wherein the emotional interface device (70) comprises a plurality of keys, and wherein each key is configured to indicate the user's emotional state, preferably by generating a different tactile and/or visual stimulus for each of the keys, and/or each of the keys of the emotional interface device (70) comprises a different tactile coating associated with a specific emotional state, and/or each of the keys of the emotional interface device (70) comprises a different colour associated with a specific emotional state and/or each key comprises a device for generating at least one stimulus taken from the stimulus or an additional stimulus different from other stimuli from the user, enabling the user to indicate their emotional state.

6. The system (1) according to one of the preceding claims, configured to, preferably via a facial recognition device of the system such as for example a camera or photographic apparatus, determine based on the user's facial recognition, at least one of the user's age, user's gender, user's emotion, ethnicity, user's temperature, user's sweating.

7. The system (1) according to any one of the preceding claims, wherein the physiological profile is generated without the user knowingly giving instructions in response to said at least two stimuli.

8. The system according to any one of the preceding claims, wherein the user fragrance (50) is selected without the user knowingly giving instructions in response to said at least two stimuli.

9. The system (1) according to any one of the preceding claims, wherein the generation of the physiological profile (31) by the system (1) from at least one physiological piece of data, and the selection by said system (1) of at least one user fragrance (50) from the database (40) based on the physiological profile (31), the user profile (20), and the piece of data relating to an objective to be achieved, are carried out by a series of algorithms forming an artificial intelligence (45).

10. The system (1) according to any one of the preceding claims, wherein the physiological profile (31) further comprises a history of the user's responses to stimuli generated and intended for reception by the user, the system being configured such that said history is generated by implementing the following steps:
- Identifying, according to the objectives to be achieved with personal data, a target profile pre-recorded in the database,
- At least one cycle, and preferably at least two cycles, each cycle comprising the following successive steps, some of at least these steps being performed by a data processing computer system:
a) Generating at least one additional stimulus,
b) Sensing at least one physiological piece of data and a brain signal derived from a brain wave of the user, the physiological piece of data and brain wave being generated by the user in response to said at least one additional stimulus,
c) Updating the physiological profile based on the physiological data and the brain wave generated by the user in response to said at least one additional stimulus,
d) Comparing the physiological profile updated with profiles pre-recorded in the database,
e) If the physiological profile actuated:
- corresponds to a profile pre-recorded in the database, then generating the history on the basis of the additional stimuli of all the cycles,
- does not correspond to any profile pre-recorded in the database, then repeating steps a to d with stimuli having different parameters from one cycle to the next.

11. A method of use of the system (1) according to any of the preceding claims to assist a user in selecting a fragrance from a database (40) including a plurality of fragrances, each including a fragrance profile, **characterised in that** the method to assist in selecting a fragrance comprises the following steps implemented by a computer system using at least one microprocessor:
a) Generating a piece of data relating to an objective to be achieved and a user profile (20) of the user, the data relating to the objective to be achieved and the user profile (20) being based on personal information provided directly or indirectly by the user;
b) Generating a preselection of fragrances especially based on the piece of data relating to an objective to be achieved, the user profile (20), and chemical characteristics of the fragrances stored in the database (40),
c) Generating at least two stimuli intended for reception by the user, said at least two stimuli being taken from olfactory, visual, and/or sound stimuli,
d) Generating a physiological profile (31) based on at least:
i. at least one brain signal, the brain signal being based on at least one brain wave generated by the user in response to said at least two stimuli;
ii. at least two physiological pieces of data of the user, different from the at least one brain wave, said at least two physiological pieces of data being sensed in response to generating and sensing at least two stimuli;
e) Determining a user fragrance profile by an algorithm based on the physiological profile (31), the user profile (20), and the piece of data relating to a objective to be achieved by the user;
f) Comparing the user fragrance profile determined by the algorithm with the fragrance profiles in the database (40) and selecting at least one user fragrance (50);
g) Displaying the at least one user fragrance (50) selected, preferably using a screen.

12. The method of use according to the preceding claim, wherein the stimuli have several parameters, preferably taken from the nature of the stimulus, a duration, and an intensity, said parameters being automatically determined based at least on said personal data provided directly or indirectly by the user and/or the physiological data and the at least one brain signal respectively sensed by the at least two physiological sensors (30) and the device for sensing the at least one brain wave, preferably, the physiological profile (31) further comprises a history of the user's responses to the stimuli generated and to be received by the user.

13. The method according to the preceding claim, wherein said history is generated by performing the following steps:
- Identifying, based on the objectives to be achieved and personal data, a target profile pre-recorded in the database,
- At least one cycle, and preferably at least two cycles, preferably at least five cycles, each cycle comprising the following successive steps, at least some of these steps being performed by a data processing computer system:
a) Generating at least one additional stimulus,
b) Sensing at least one physiological piece of data and a brain signal derived from a brain wave of the user, the physiological piece of data and brain wave being generated by the user in response to said at least one additional stimulus,
c) Updating the physiological profile based on the physiological data and the brain wave generated by the user in response to said at least one additional stimulus,
d) Comparing the physiological profile updated with profiles pre-recorded in the database,
e) If the physiological profile actuated:
- corresponds to a profile pre-recorded in the database, then generating the history on the basis of the additional stimuli of all the cycles,
- does not correspond to any pre-recorded profile in the database, then repeating steps a to d with stimuli having different parameters from one cycle to the next, preferably, the user does not knowingly interact with the system upon generating the physiological profile (31).

14. A booth (60) comprising a system (1) according to one of claims 1 to 10, comprising a neuro headset (36) configured to detect at least one brain wave, a user interface (10), a screen (32), and at least the physiological sensor (30) configured to sense the at least two different physiological pieces of data, different from the at least one brain wave sensed by the neuro headset (36).

15. The booth (60) according to the preceding claim comprising a system for diffusion of a perfume by dry diffusion.
